# EUROPEAN PATENT SPECIFICATION

(11) **EP 1 553 989 B1**
(45) Date of publication and mention of the grant of the patent: **22.03.2006**
(21) Application number: 03792228.3
(22) Date of filing: 24.07.2003
(51) Int. Cl.: A61L 15/18, A61L 15/60, B01J 20/26, B01J 20/12

(54) **SUPERABSORBENT POLYMERS AND METHOD OF MANUFACTURING THE SAME**
SUPERSAUGFÄHIGE POLYMERE UND VERFAHREN ZU IHRER HERSTELLUNG
POLYMERES SUPERABSORBANTS ET PROCEDE DE PRODUCTION CORRESPONDANT

(30) Priority: 23.08.2002 US 405783 P
(43) Date of publication of application: 20.07.2005
(73) Proprietor: BASF Aktiengesellschaft, 67056 Ludwigshafen (DE)
(72) Inventor: HERFERT, Norbert, Charlotte, NC 28226-7200 (US); MITCHELL, Michael A., Waxhaw, NC 28173 (US); AZAD, Michael M., Charlotte, NC 28277 (US); WOODRUM, Guy T., Suffolk, VA 23435 (US); CHIANG, William G-J, Yorktown, VA 23692 (US)
(86) International application number: PCT/EP2003/008092
(87) International publication number: WO 2004/018006

(56) References cited:
- WO-A-01/13965
- WO-A-01/68156
- WO-A-96/30442
- US-A- 5 140 076
- US-A- 6 124 391

## Description

### FIELD OF THE INVENTION

The present invention relates to surface-crosslinked superabsorbent polymers (SAPs) and to an improved method of manufacturing the SAPs. More particularly, the present invention relates to the incorporation of a clay into SAP particles during a surface crosslinking step. The resulting surface crosslinked SAP particles exhibit excellent fluid absorption and retention properties, especially with respect to permeability of a fluid through a bed of the surface crosslinked SAP particles. The incorporation of a clay also provides a more facile and economical method of preparing SAP particles.

### BACKGROUND OF THE INVENTION

Water-absorbing resins are widely used in sanitary and hygienic goods, wiping cloths, water-retaining agents, dehydrating agents, sludge coagulants, disposable towels and bath mats, disposable door mats, thickening agents, disposable litter mats for pets, condensation-preventing agents, and release control agents for various chemicals. Water-absorbing resins are available in a variety of chemical forms, including substituted and unsubstituted natural and synthetic polymers, such as hydrolysis products of starch acrylonitrile graft polymers, carboxymethylcellulose, crosslinked polyacrylates, crosslinked and partially neutralized copolymers of isobutylene and maleic anhydride, saponification products of vinyl acetate-acrylic acid copolymer, sulfonated polystyrenes, hydrolyzed polyacrylamides, polyvinyl alcohols, polyethylene oxides, polyvinylpyrrolidones, and polyacrylonitriles.

These polymers, and others, are known in the art by various names, such as superabsorbent polymers, hydrogels, hydrocolloids, and water-absorbent hydrophilic polymers, for example. As used herein, the term "SAP" refers to a superabsorbent polymer, and collectively refers to such water-absorbing materials. As used herein, the term "SAP particles" refers to superabsorbent polymer particles in the dry state, more specifically, particles containing from no water up to an amount of water less than the weight of the particles, and typically less than about 5%, by weight, water. The term "particles" refers to granules, fibers, flakes, spheres, powders, platelets, and other shapes and forms known to persons skilled in the art of superabsorbent polymers. The terms "SAP gel," "SAP hydrogel," or "hydrogel" refer to a superabsorbent polymer in the hydrated state, more specifically, particles that have absorbed at least their weight in water, and typically several times their weight in water. The terms "surface-treated SAP particle" and "surface-crosslinked SAP particle" refer to an SAP particle having its molecular chains present in the vicinity of the particle surface crosslinked by a polyfunctional compound applied to the surface of the particle. The term "surface crosslinking" means that the level of functional crosslinks in the SAP particle in the vicinity of the surface of the particle is generally higher than the level of functional crosslinks in the SAP particle in the interior of the particle.

SAPs are lightly crosslinked hydrophilic polymers, and are discussed generally in U.S. Patent Nos. 5,669,894 and 5,559,335, each incorporated herein by reference. SAPs can differ in their chemical identity, but all SAPs are capable of absorbing and retaining amounts of aqueous fluids equivalent to many times their own weight, even under moderate pressure. For example, SAPs can absorb one hundred times their own weight, or more, of distilled water. The ability to absorb aqueous fluids under a confining pressure is an important requirement for an SAP used in a hygienic article, such as a diaper.

An SAP, like poly(acrylic acid), typically is neutralized at least about 25 mole percent, preferably at least about 50 mole percent, and usually about 70 to 80 mole percent, to achieve optimum absorbency. Neutralization can be achieved by neutralizing the acrylic acid monorner before polymerization of the monomer, or the polymer can be neutralized after the polymerization reaction is substantially complete. After polymerization and internal crosslinking of the monomer, followed by partial neutralization, the resulting hydrogel is subdivided, e.g., shredded or chopped, for more efficient processing, then dried and milled to a desired particle size. The SAP particles preferably then are surface treated and dried again to form the final product.

Many improvements have been made in the performance and properties of SAPs, such as in gel strength and reabsorbing capacity. However, improvements in the manufacturing processes for SAPs have been relatively neglected. SAP manufacturing process improvements are important because the SAP is an expensive element in an absorbent article, like a diaper. Thus, reducing the cost of an SAP by a process improvement has a direct effect on the cost of an article incorporating the SAP. An important process improvement would be an increase in the rate of SAP manufacture, thereby increasing the amount of SAP prepared in a given time period.

One problem encountered in the production of SAP particles is the generation of fine-sized particles that must be sifted from the SAP particles prior to shipment. These fine-sized SAP particles, i.e., SAP fines, then are recycled into the SAP production process so that they are not wasted. However, the recycling of SAP fines adds an extra step to the SAP manufacturing process, and is time consuming and expensive because the recycled SAP fines are dried twice.

In the SAP production process, the SAP hydrogel drying step is the rate limiting step in the production of an SAP. In a typical example, when an SAP hydrogel is chopped to a small particle size, the SAP hydrogel solids can be increased by the addition of dry SAP fines, such that the hydrogel contains about 30% to 32% polymer and 68% to 70% water, by weight. Thus, an SAP production process that reduces or avoids the generation and recycling of SAP fines would provide an improved SAP manufacturing process.

Therefore, it would be desirable to provide a method of increasing the production rate of an SAP by reducing, or essentially eliminating, the production of fine-sized SAP particles. The improved method preferably substantially eliminates the step of reintroducing SAP fines to an SAP hydrogel, and substantially eliminates drying of a significant portion of the SAP hydrogel a second time. The improved SAP production process would provide a savings in SAP production time and in energy costs. The improved method also must not adversely affect the ability of the dried SAP particles to absorb fluids quickly, to demonstrate good fluid permeability and conductivity into and through the SAP particles, and to resist SAP deformation or flow under an applied stress or pressure.

Accordingly, the present invention is directed to an improved method of manufacturing SAP particles comprising the addition of a clay to SAP particles during a surface crosslinking step. It has been found that the addition of a clay to SAP particles during the surface crosslinking step significantly reduces the amount of SAP fines, thus providing increased SAP production rates.

The surface treatment of SAP particles is well known. Surface crosslinked SAP particles, in general, exhibit higher liquid absorption and retention values than SAP particles having a comparable level of internal crosslinks, but lacking surface crosslinking. As understood in the art, surface crosslinked SAP particles have a higher level of crosslinking in the vicinity of the surface than in the interior. As used herein, "surface" describes the outer-facing boundaries of the particle. For porous SAP particles, exposed internal surfaces also are included in the definition of surface. Surface crosslinking of SAPs is generally discussed in F.L Buchholz et al., ed., "Modern Superabsorbent Polymer Technology," Wiley-VCH, New York, NY, pages 97-108 (1998).

In addition to surface crosslinking, clays and other mineral products have been added to SAPs in an attempt to improve SAP performance. For example, the addition of finely divided amorphous silica, such as AEROSIL®, available from Degussa, DE, or CAB-O-SIL®, available from Cabot Corporation, or a bentonite onto the surface of SAP powders or granules is known. U.S. Patent Nos. 5,140,076 and 4,734,478 disclose the addition of silica during surface crosslinking of dry SAP powders. U.S. Patent No. 4,286,082 discloses mixtures of silica and an SAP for use in hygiene articles.

JP 65 133 028A and JP 61 017 542B disclose mixtures of hydrophobic silicas and absorbent polymers. EP 0 341 951, U.S. Patent No. 4,990,338, and U.S. Patent No. 5,035,892 disclose the use of silica in the production of antimicrobial absorbent polymers. U.S. Patent No. 4,535,098 and EP 0 227 666 disclose the addition of silica-based colloidal substances to enhance the gel strength of SAPs.

Generally, in mixtures of dry SAP particles with a silica powder, the silica adheres to the SAP particle surfaces and alters the surface properties of the SAP particles, but not their intrinsic absorption properties. For example, the silica powder is hydrophilic or hydrophobic, which primarily influences the rate at which a fluid is absorbed by the SAP particles.

WO 99/64515 discloses the preparation of SAPs by polymerizing olefinically unsaturated carboxylic acids and adding a silicate before, during, and after polymerization. The swollen polymer particles have improved mechanical stability and enhanced permeability. However, because the silicate framework lacks a charge, no osmotic pressure can be generated. This neutral silicate framework does not contribute to the osmotic swell pressure of the hydrogel, and fluid absorbency is adversely effected.

WO 99/55767 discloses ionically crosslinked SAPs obtained by polymerizing carboxyl-containing monomers and adding aluminate ions before, during, and after polymerization. The presence of ionic crosslinked sites provides improved gel stability under mechanical load. However, the salt stability of these hydrogels is inadequate, and a premature collapse of the network structure occurs at a high salt content.

Other patents and applications disclosing SAP particles and a clay include GB 2,082,614 disclosing a dry, solid, water-swellable absorbent composition prepared by blending dry SAP particles and 1% to 75%, by weight of the blend, of an extender material selected from uncrosslinked cellulose derivatives, starch, certain clays and minerals, and mixtures thereof.

U.S. Patent No. 4,500,670 discloses water-absorbent compositions containing a water-swellable SAP and an inorganic powder, preferably clay. The compositions are prepared by physically blending the inorganic powder and SAP particles after the SAP is polymerized and crosslinked.

U.S. Patent No. 4,735,987 discloses polymerization of a partially neutralized acrylic acid via an inverse suspension polymerization process, with the simultaneous addition of a crosslinker and an inorganic material, e.g., a clay, to the polymer bead suspension, followed by azeotropic dehydration. Crosslinking occurs in the presence of the clay during the dehydration step. The resulting product has a high volume expansion after swelling in saline solution.

U.S. Patent No. 4,914,066 discloses pellets containing 0.5 to 15 wt.% SAP and 85 to 99.5 wt.% bentonite clay, prepared by mixing the SAP and bentonite in the presence of water, then compressing and extruding the blend through an orifice to form the pellets, followed by drying.

WO 91/12029 and WO 91/12031 disclose compositions containing an SAP combined with odor-controlling agents, preferably zeolites, by means of a binder. The SAP particles are coated with the zeolite in presence of a binder in a fluidized bed coating apparatus, or are admixed with dry SAP particles and water, and the mixture is dried by heating.

U.S. Patent No. 5,419,956 discloses mixtures of SAP fines with an inorganic powder, like silica or clay.

U.S. Patent No. 5,733,576 discloses a process of producing absorbing agents containing (a) a water-swellable, synthetic polymer or copolymer, and (b) a natural or synthetic polymeric compound which at normal temperature is a pourable powder and is partially soluble or insoluble in water. The absorbing agents can contain clay as a neutral filling agent.

EP 0 799 861 discloses a particulate deodorant composition containing an SAP and powdery zeolite dispersed within the SAP resin particles. The composition is prepared by kneading a water-absorbent resin and a zeolite powder in the presence of water, followed by drying and grinding.

U.S. Patent No. 6,124,391 discloses SAP particles containing 0.2 to 10 wt% of an inorganic powder, e.g., clay, having improved anticaking properties, wherein more than 60 wt% of the particles are larger than 300 µm. Clay is added before, during, or after a surface crosslinking step.

WO 00/72958 discloses a process for producing a networked polymer/clay alloy for use in a personal care article. The process comprises the steps of:
(a) preparing a monomer/clay mixture by mixing at least a monomer, clay particles, a crosslinking agent, and a mixing fluid in a vessel;
(b) exposing the monomer/clay mixture to a polymerization initiator; and
(c) polymerizing the monomer/clay mixture to form a networked polymer/clay alloy.

WO 01/13965 discloses water-absorbent polymers containing silicium-rich zeolites for odor control. The silicium-rich zeolites can be added to the monomer solution, to the SAP gel, or in the surface crosslinking step.

WO 01/32117 discloses an SAP composition containing a partially neutralized SAP wherein at least 30% of the functional groups of the polymer are in free acid form, and a layered double hydroxide anionic clay, e.g., hydrotalcite clays. In the examples, the composites were prepared by powder/powder mixing.

WO 01/68156 discloses a hydrophilic swellable hydrogel-forming polymer containing aluminosilicate and having enhanced permeability and improved odor-control properties. The aluminosilicates can be added before, during, or after polymerization.

However, a need still exists for an SAP manufacturing process that reduces or essentially eliminates the generation of SAP fines, and, accordingly, the costly and time-consuming steps involved in recycling the SAP fines. The resulting SAP particles also must possess the fluid absorption and retention properties required of SAP particles incorporated into an absorbent article. The above-described compositions containing an SAP and an inorganic material, like a clay, have not met these needs.

Accordingly, the present invention is directed to a method of improving the manufacture of SAP particles by introducing a clay to SAP particles during a surface crosslinking step. The addition of a clay to SAP particles during surface crosslinking significantly reduces the generation of SAP fines, without adversely affecting SAP performance with respect to (a) fluid absorption and retention by the SAP particles, and (b) the rate of fluid absorption and fluid permeability through fluid swollen SAP particles.

### SUMMARY OF THE INVENTION

The present invention is directed to surface crosslinked SAP particles and methods of manufacturing the SAP particles. More particularly, the present invention is directed to SAP particles comprising a water-absorbing resin and a clay, and a method of manufacturing such SAP particles, wherein the clay is added to the SAP particles during a surface crosslinking step. The clay is present in the vicinity of the surfaces of the SAP particles.

One aspect of the present invention, therefore, is to provide a method of manufacturing SAP particles including the steps of polymerizing an aqueous monomer mixture containing an α,β-unsaturated carboxylic acid, such as acrylic acid, either neutralized, unneutralized, or a mixture thereof (i.e., DN (degree of neutralization) of 0 to 100, and preferably about 50 to about 80), to form an SAP hydrogel, comminuting the SAP hydrogel to a desired particle size, and, if necessary or desired, neutralizing the SAP hydrogel, then drying the SAP hydrogel particles to provide the SAP particles. The SAP particles next are subjected to a surface crosslinking step by treatment with a surface crosslinking agent in the presence of a clay. This process positions the clay in the vicinity of the surfaces of the SAP particles.

Another aspect of the present invention is to provide a method of manufacturing SAP particles by a solution polymerization process comprising: (a) forming an aqueous monomer solution comprising (i) one or more monomers capable of providing an SAP, like an α,β-unsaturated carboxylic acid, such as acrylic acid, (ii) a polyethylenically unsaturated polymerizable monomer as an internal crosslinking monomer, and (iii) a redox catalyst system and/or a thermal free radical initiator; (b) polymerizing the monomers in the monomer solution a sufficient amount to form an SAP hydrogel having a free monomer content of less than 1000 ppm (parts per million); (c) comminuting the SAP hydrogel, e.g., extruding or gel chopping, to provide SAP hydrogel particles of desired particle size; (d) drying the SAP hydrogel particles to provide SAP particles; and (e) surface crosslinking the SAP particles in the presence of a clay.

Yet another aspect of the present invention is to provide a method of manufacturing SAP particles comprising the addition of a sufficient amount of a clay to SAP particles in a surface crosslinking step to reduce or essentially eliminate the generation and recycling of SAP fines.

Another aspect of the present invention is to provide a method of manufacturing SAP particles wherein a clay is incorporated into the SAP particles in a surface crosslinking step to substantially reduce unit processing time for the manufacture of SAP particles and to reduce operation costs.

Another aspect of the present invention is to provide surface-treated SAP particles comprising about 0.001 % to about 5% by weight of a surface crosslinking agent and about 12% to about 30% parts by weight of a clay, each by weight of SAP particles, and applied to the surfaces of the SAP particles to crosslink the molecular chains existing at least in the vicinity of the surfaces of the SAP particles, and to incorporate a clay into the SAP particles at least in the vicinity of the surfaces of the SAP particles.

Yet another aspect of the present invention is to provide a method of manufacturing SAP particles that reduces or essentially eliminates the generation of SAP fines, i.e., SAP particles having a diameter of less than about 200 µM. This important feature eliminates the need to sift SAP fines from the SAP, and reduces or essentially eliminates recycling and redrying of the SAP fines in an SAP hydrogel.

Still another aspect of the present invention is to provide surface-crosslinked SAP particles comprising a water-absorbing resin, up to 25%, by weight, of an inorganic network builder, e.g., a silicate, distributed throughout the particle, and about 12% to about 35%, by weight, of a clay distributed in the vicinity of the surfaces of the SAP particles, wherein the surface-crosslinked SAP particles exhibit excellent fluid absorption, retention, and permeability properties.

Another aspect of the present invention is to provide SAP particles containing an SAP and a clay distributed in the vicinity of the surfaces of the particles. The particles have high absorbance and retention properties for aqueous fluids, a fast fluid acquisition rate and an excellent fluid permeability in the swollen state, and maintain a "dry feel" after significant liquid absorption.

Still another aspect of the present invention is to provide a composition comprising SAP particles having a clay in the vicinity of the surfaces thereof, wherein the clay is selected from the group consisting of (a) a swelling clay, (b) a nonswelling clay, (c) mixtures thereof, wherein the clay is incorporated into the SAP particles during a surface crosslinking step.

In preferred embodiments of the present invention, the SAP particles comprise a partially neutralized SAP, e.g., poly(acrylic acid) (PAA) or a poly(vinylamine) (PVAm), containing at least 25%, and up to 100%, neutralized carboxyl groups or amino groups, and a nonswellable clay.

Yet another aspect of the present invention is to provide articles of manufacture containing the surface crosslinked SAP particles of the present invention, for example, a diaper, a catamenial device, a feminine hygiene product, an adult incontinence product, general purpose wipes and cloths, and similar absorbent products.

Further aspects and advantages of the present invention will become apparent to those skilled in the art from the following detailed description of the preferred embodiments, taken in conjunction with the examples and the appended claims.

### DETAILED DESCRIPTION OF THE PREFERRED EMBODIMENTS

The present invention is directed to absorbent particles comprising (a) a surface-crosslinked SAP and (b) a clay. The SAP and clay are present in a single particle, as opposed to an admixture of discrete SAP particles and discrete clay particles. The clay is introduced to the SAP particles during surface crosslinking of the SAP particles, and is present in the vicinity of the surfaces of the SAP particles.

In accordance with an important feature of the present invention, the SAP particles contain about 50% to about 88%, and preferably about 55% to about 85%, by weight, of the surface-crosslinked SAP. To achieve the full advantage of the present invention, the particles contains about 60% to about 85%, by weight, of the surface-crosslinked SAP. The SAP particles contain about 12% to about 35%, and preferably about 15% to about 25%, by weight, of the clay. To achieve the full advantage of the present invention, the composition contains about 15% to about 20%, by weight, of the clay. The surface-crosslinked SAP particles optionally can contain up to about 25%, by weight, of an inorganic network builder, such as a silicate, like sodium silicate.

The water-absorbing resin component of the present SAP particles is prepared by well-known continuous and discontinuous processes. The monomers comprising the water-absorbing resin component of the SAP particles typically are polymerized in aqueous solution to form an SAP hydrogel. However, the water-absorbing resin component of the present particles can be prepared by any other method known to persons skilled in the art, like inverse suspension polymerization.

The monomers of the SAP comprise an ethylenic monomer having a carboxylic acid substituent or a precursor to a carboxylic acid substituent, e.g., an α,β-unsaturated carboxylic acid or anhydride thereof, typically acrylic acid, or acrylonitrile or a (meth)acrylamide, or an ethylenic monomer having an amine substituent or a precursor to an amine substituent, e.g., N-vinyl acetamide. The monomers used in the polymerization are unneutralized or neutralized, i.e., contain 0% to 100% of the carboxyl or amino groups in the free acid or free base form, respectively. The product of the polymerization process is an SAP hydrogel. Generally, the SAP hydrogel is subjected to a mechanical comminution, i.e., reduction of the particle size of the SAP hydrogel, for example, by chopping. Then, the SAP hydrogel particles are dried to remove water and provide dry SAP particles. The dry SAP particles optionally can be subjected to further mechanical means for particle size reduction and classification including chopping, grinding, and sieving. The surface crosslinking agent is applied to the dried SAP particles. Generally, after application of the surface crosslinking agent, the SAP particles are subjected to conditions wherein the surface crosslinking agent reacts with a portion of the carboxyl or amino groups of the SAP to crosslink the surfaces of the SAP particles.

In one method of manufacturing SAP particles, the monomers, including an α,β-unsaturated carboxylic acid, neutralized or unneutralized, are added as an aqueous solution to an unagitated vertical reactor. The aqueous monomer solution can further include optional ingredients, and typically includes an internal crosslinking agent to render the resulting polymer water insoluble. The addition of suitable initiators under suitable reaction conditions leads to polymerization of the monomers and internal crosslinking agent to form an SAP hydrogel. In such procedures, the aqueous monomer solution generally is maintained as a single-phase system until solid particles of polymer are formed.

The SAP hydrogel resulting from the solution polymerization is a stiff gel, typically containing about 26% by weight of polymer. The remaining portion of the SAP hydrogel is essentially completely water. The SAP hydrogel then is subjected to a comminution step wherein the SAP hydrogel is chopped or extruded to provide an SAP hydrogel of the desired particle size distribution. Typically, during the gel chopping step, dry SAP fines are recycled into the SAP hydrogel to increase the amount of polymer in the SAP hydrogel to about 30% to 32% by weight prior to the drying step. If necessary or desired, the SAP hydrogel can be neutralized to a predetermined degree of neutralization during the gel chopping step. After the gel chopping step, the SAP hydrogel contains about 68% to 70%, by weight, water, which must be removed in a drying step to provide the dry SAP particles. The dry SAP particles then are surface crosslinked.

The chopping and grinding of the SAP particles generates about 20% to about 25% of SAP fines, i.e., SAP particles having a diameter of less than 200 µm. Such SAP fines are unsuitable for use in absorbent articles, but are too expensive to discard. Therefore, the SAP fines are recycled into an SAP hydrogel, in small amounts as discussed above. However, each SAP batch provides additional SAP fines, so the problem is ongoing.

The problem of SAP fines is costly in terms of adding recycling steps to SAP manufacture, including sifting of the SAP particles, reintroduction of SAP fines that have been dried once to an SAP hydrogel, then drying a portion of the SAP a second time. The generation of SAP fines adversely affects the production rate of an SAP because the rate-limiting step in SAP manufacture is drying, and recycling of once-dried SAP fines requires drying a portion of the SAP particles a second time.

Accordingly, a method of manufacturing an SAP that reduces or essentially eliminates the generation of SAP fines would be a substantial advance in the art. The present invention is directed to such a method.

More particularly, it has been found that adding a clay to SAP particles during a surface crosslinking step reduces or essentially eliminates the generation of SAP fines, thereby eliminating the SAP fines recycling step, or at least substantially reducing the scope of the recycling step. The addition of a clay during a surface cross-linking step does not adversely affect SAP performance, but improves various properties, such as SAP particle permeability to fluids.

The surface crosslinking step typically is performed immediately after the SAP particles are synthesized, dried to an appropriate water content, and sized. In accordance with an important feature of the present invention, a clay is incorporated into the SAP particles during the surface crosslinking step. The amount of clay incorporated onto the SAP particles is about 12% to about 35%, by weight, based on the weight of the SAP particles. It has been demonstrated that incorporating a clay onto the surfaces of SAP particles decreases the amount of SAP fines by about 70% to about 95%, and typically by about 75% to about 90%.

In accordance with the present invention, SAP particles resulting from monomer polymerization, including SAP fines, are surface crosslinked in the presence of a clay, typically as an aqueous clay slurry. Surface crosslinked SAP particles of the present invention have a clay distributed in the vicinity of the SAP particle surfaces. The surface crosslinked SAP particles can be prepared, for example, by a method comprising the steps of polymerizing at least one vinyl monomer capable of providing an SAP, e.g., an α,β-unsaturated carboxylic acid, in its neutralized and/or unneutralized form, to form an SAP hydrogel, drying the SAP hydrogel to form SAP particles, applying a mixture comprising a clay and a surface crosslinking agent to the surfaces of the SAP particles, then heating the surface treated SAP particles to provide surface crosslinks and position the clay in the vicinity of the surfaces of the SAP particles.

The remainder of the specification is particularly directed to an SAP based on acrylic acid. However, other vinyl monomers (e.g., vinyl amine and its precursors) and other α,β-unsaturated carboxylic acids, anhydrides, and carboxylic acid precursors can be employed to prepare an SAP having the ability to absorb several times its weight of an aqueous liquid, and useful in the absorbent particles of the present invention. In particular, surface crosslinked SAP particles of the present invention, and their method of manufacture, reduce or essentially eliminate the generation of SAP fines, and, accordingly, the time and expense associated with recycling SAP fines, independent of the identity of the vinyl monomer and/or α,β-unsaturated carboxylic acid used to prepare the SAP. The present surface crosslinked SAP particles also exhibit an improved fluid permeability through the fluid swollen particles, and have an improved dry feel of fluid swollen particles.

In use, the surface crosslinked SAP particles of the present invention can be used in absorbent articles, such as diapers, catamenial devices, and adult incontinence products. The particles are especially useful in absorbing electrolyte-containing fluids, such as urine, menses, and blood.

Various embodiments of the present invention, and a nonlimiting description of the components of the present surface crosslinked SAP particles, follow.

### SUPERABSORBENT POLYMER (SAP)

An SAP used in the surface crosslinked SAP particles of the present invention is limited only in that the SAP is capable of absorbing several times its weight of an aqueous fluid and swells to form a hydrogel. The SAP can be an acidic water-absorbing resin or a basic water-absorbing resin. Monomers useful in the preparation of an SAP are disclosed in U.S. Patent No. 5,149,750 and WO 01/68156, each incorporated herein by reference. The SAP component of the present SAP particles comprises an acidic or a basic water-absorbing resin neutralized about 25% to about 100%, i.e., has a degree of neutralization (DN) of about 25 to about 100.

The SAP can be anionic (an acidic water-absorbing resin) of cationic (a basic water-absorbing rein) in nature. The anionic SAPs are based on an acidic water-absorbing resin. The anionic SAPs, either strongly acidic or weakly acidic, can be any resin that acts as an SAP in its neutralized form. The acidic resins typically contain a plurality of carboxylic acid, sulfonic acid, phosphonic acid, phosphoric acid, and/or sulfuric acid moieties.

A preferred SAP is an acidic water-absorbing resin neutralized 25% to 100%. The acidic water-absorbing resin can be a single resin or a mixture of resins. The acidic resin can be a homo-polymer or a copolymer. The identity of the acidic water-absorbing resin is not limited as long as the resin is capable of swelling and absorbing at least ten times its weight in water, when in a neutralized form.

The acidic water-absorbing resin typically is a lightly crosslinked acrylic resin, such as lightly crosslinked poly(acrylic acid). The lightly crosslinked acidic resin typically is prepared by polymerizing an acidic monomer containing an acyl moiety, e.g., acrylic acid, or a moiety capable of providing an acid group, e.g., a (meth)acrylamide, an alkyl (meth)acrylate, or acrylonitrile, in the presence of an internal crosslinking monomer, i.e., a polyfunctional organic compound. The acidic resin can contain other copolymerizable units, i.e., other monoethylenically unsaturated comonomers, well known in the art, as long as the polymer is substantially, i.e., at least 10%, and preferably at least 25%, acidic monomer units. To achieve the full advantage of the present invention, the acidic resin contains at least 50%, and more preferably, at least 75%, and up to 100%, acidic monomer units.

Ethylenically unsaturated carboxylic acid and carboxylic acid anhydride monomers useful in the acidic water-absorbing resin include acrylic acid, methacrylic acid, ethacrylic acid, α-chloroacrylic acid, α-cyanoacrylic acid, β-methylacrylic acid (crotonic acid), α-phenylacrylic acid, β-acryloxy-propionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, furmaric acid, tricarboxyethylene, and maleic anhydride. Acrylic acid is the most preferred ethylenically unsaturated carboxylic acid for preparing the SAP.

Ethylenically unsaturated sulfonic acid monomers include aliphatic and aromatic vinyl sulfonic acids, such as vinyl sulfonic acid, allyl sulfonic acid, vinyl toluene sulfonic acid, styrene sulfonic acid, acrylic and methacrylic sulfonic acids, such as sulfoethyl acrylate, sulfoethyl meth-acrylate, sulfopropyl acrylate, sulfopropyl methacrylate, 2-hydroxy-3-methacryloxypropyl sulfonic acid, and 2-acrylamido-2-methylpropane sulfonic acid. Phosphate-containing acidic resins are prepared by homopolymerizing or copolymerizing ethylenically unsaturated monomers containing a phosphoric acid moiety, such as methacryloxy ethyl phosphate. An extensive list of suitable SAP-forming monomers can be found in U.S. Patent No. 4,076,663, incorporated herein by reference.

The anionic SAPs can be, for example, a poly(acrylic acid), a hydrolyzed starch-acrylonitrile graft copolymer, a starch-acrylic acid graft copolymer, a saponified vinyl acetate-acrylic ester copolymer, a hydrolyzed acrylonitrile copolymer, a hydrolyzed acrylamide copolymer, an ethylene-maleic anhydride copolymer, an isobutylene-maleic anhydride copolymer, a poly(vinylsulfonic acid), a poly(vinyl-phosphonic acid), a poly(vinylphosphoric acid), a poly(vinylsulfuric acid), a sulfonated polystyrene, and mixtures thereof. The preferred anionic SAP is a poly(acrylic acid).

The polymerization of acidic monomers, and copolymerizable monomers, if present, most commonly is performed by free radical processes in the presence of a polyfunctional internal crosslinking monomer. The acidic resins are crosslinked to a sufficient extent such that the polymer is water insoluble. Crosslinking renders the acidic resins substantially water insoluble, and, in part, serves to determine the absorption capacity of the resins. For use in absorption applications, an acidic resin is lightly crosslinked, i.e., has a crosslinking density of less than about 20%, preferably less than about 10%, and most preferably about 0.01 % to about 7%.

An internal crosslinking monomer most preferably is used in an amount of less than about 7 wt%, and typically about 0.1 wt% to about 5 wt%, based on the total weight of monomers. Examples of internal crosslinking monomers include, but are not limited to, poly(meth)acrylic acid esters represented by the following formula (I), 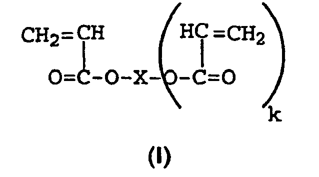 wherein x is ethylene, propylene, trimethylene, cyclohexyl, hexamethylene, 2-hydroxypropylene, -(CH₂CH₂O)ₙCH₂CH₂-, or 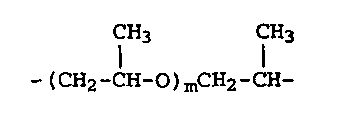 n and m, independently, are an integer 5 to 40; and k is 1 or 2; and bisacrylamides, repre-sented by the following formula (II),

CH₂=CH-C(=O)-NH(CH₂CH₂NH)₁C(=O)-CH=CH₂ (II)

wherein l is 2 or 3.

The compounds of formula (I) are prepared by reacting polyols, such as ethylene glycol, propylene glycol, trimethylolpropane, 1,6-hexanediol, glycerin, pentaerythritol, polyethylene glycol, or polypropylene glycol, with acrylic acid or methacrylic acid. The compounds of formula (II) are obtained by reacting polyalkylene polyamines, such as diethylenetriamine and triethylenetetramine, with acrylic acid. Specific crosslinking monomers are disclosed in U.S. Patent No. 6,222,091, incorporated herein by reference. Especially preferred crosslinking agents are pentaerythritol triallyl ether, N,N'-methylenebisacrylamide, N,N'-methylenebismethacrylamide, ethylene glycol dimethacrylate, and trimethylolpropane triacrylate.

Analogous to the acidic resin, a basic water-absorbing resin, i.e., cationic SAP, useful in the present SAP particles can be a strong or weak basic water-absorbing resin. The basic resin, either strongly or weakly basic, therefore, can be any resin that acts as an SAP in its charged form. The basic water-absorbing resin can be a single resin or a mixture of resins. The basic resin can be a homopolymer or a copolymer. The identity of the basic resin is not limited as long as the basic resin is capable of swelling and absorbing at least 10 times its weight in water, when in a charged form. The weak basic resin preferably is present in its cationic form, i.e., about 25% to 100% of the basic moieties, e.g., amino groups, are present in a charged form. The strong basic resins typically are present in the hydroxide (OH) or bicarbonate (HCO₃) form.

The basic water-absorbing resin typically is a lightly crosslinked resin, such as a poly(vin-ylamine) or a poly(dialkylaminoalkyl (meth)acryl-amide). The basic resin also can be, for example, a lightly crosslinked polyethylenimine, a poly(allylamine), a poly(allylguanidine), a poly(dimethyldiallylammonium hydroxide), a quaternized poly-styrene derivative, a guanidine-modified polystyrene, a quaternized poly((meth)acrylamide) or ester analog. See U.S. Patent No. 6,235,965, incorporated herein by reference. The lightly crosslinked basic water-absorbing resin can contain other copolymerizable units and is crosslinked using a polyfunctional organic compound, as set forth above with respect to the acidic water-absorbing resin. Preferred basic resins include a poly(vinylamine), polyethylenimine, poly(vinylguanidine), poly(dimethylaminoethyl acrylamide) (poly(DAEA)), and poly(dimethylaminopropyl methacrylamide) (poly(DMAPMA)).

A basic water-absorbing resin used in the present SAP particles typically contains an amino or a guanidino group. Accordingly, a water-soluble basic resin also can be crosslinked in solution by suspending or dissolving an uncrosslinked basic resin in an aqueous or alcoholic medium, then adding a di- or polyfunctional compound capable of cross-linking the basic resin by reaction with the amino groups of the basic resin. Such crosslinking agents are disclosed in U.S. Patent No. 6,235,956, incorporated herein by reference. Crosslinking agents also are disclosed in Pinschmidt, Jr. et al. U.S. Patent No. 5,085,787, incorporated herein by reference, and in EP 450 923. Preferred crosslinking agents are ethylene glycol diglycidyl ether (EGDGE), a water-soluble diglycidyl ether, and a dibromoalkane, an alcohol-soluble compound.

Copolymerizable monomers for introduction into the acidic resin, or into the basic resin, include, but are not limited to, ethylene, propylene, isobutylene, C₁₋₄alkyl acrylates and methacrylates, vinyl acetate, methyl vinyl ether, and styrenic compounds having the formula: 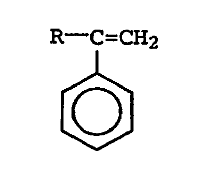 wherein R represents hydrogen or a C₁₋₆alkyl group, and wherein the phenyl ring optionally is substituted with one to four C₁₋₄alkyl or hydroxy groups.

Suitable C₁₋₄alkyl acrylates include, but are not limited to, methyl acrylate, ethyl acrylate, isopropyl acrylate, n-propyl acrylate, n-butyl acrylate, and the like, and mixtures thereof. Suitable C₁₋₄alkyl methacrylates include, but are not limited to, methyl methacrylate, ethyl methacrylate, isopropyl methacrylate, n-propylmethylmethacrylate, n-butyl methacrylate, and the like, and mixtures thereof or with C₁₋₄alkyl acrylates. Suitable styrenic compounds include, but are not limited to, styrene, α-methylstyrene, p-methylstyrene, t-butyl styrene, and the like, and mixtures thereof or with C₁₋₄alkyl acrylates and/or methacrylates.

In addition, surface-crosslinked SAP particles of the present invention can contain 0% to about 25%, preferably about 5% to about 20%, by weight, of an inorganic network builder, including, but not limited to, a silicate, like sodium silicate, an aluminate, like sodium aluminate, or an aluminosilicate. See WO 01/68156, incorporated herein by reference, for a description of aluminosilicates:

Any polymerization initiator known for use in preparing SAPs can be used. Examples of useful initiators are redox and thermal initiators, such as those disclosed in U.S. Patent No. 6,359,049, incorporated herein by reference. Redox and thermal initiators can be used singly or in suitable combination. Of these, especially preferred initiators are a redox initiator comprising ammonium persulfate and sodium hydrogen sulfite, and azo initiators, such as azobisisobutyronitrile and 2,2'-azobis(2-amidinopropane)di-hydrochloride, commercially available under the tradename V-50 from Wako Chemicals U.S.A., Inc., Richmond, Virginia. The initiator typically is used in an amount, calculated as solids, of about 0.1 % to about 10%, based on the weight of the acrylic acid monomer, preferably about 0.5% to about 5%, based on the weight of the monomer. Depending on the amount and kind of the initiator, the initiator optionally can be used together with isopropyl alcohol, an alkyl mercaptan, or other chain transfer agent to control the molecular weight of the poly(acrylic acid).

Ultraviolet (UV) light also can be used to effect polymerization of acrylic acid. UV light can be used in conjunction with a redox initiator and/or a free radical initiator. When UV light is utilized in the polymerization step, a photoinitiator also is added to the reaction mixture in an amount well known to persons skilled in the art. Suitable photoinitiators include, but are not limited to, 2-hydroxy-1-[4-(hydroxyethyoxy)phenyl]-2-methyl-1-propanone, which is commercially available from Ciba Additives of Hawthorne, New York, as IRGACURE® 2959, and 2-hydroxy-2-methyl-1-phenyl-1-propanone, which also is commercially available from Ciba Additives as DAROCUR® 1173.

Industrial processes useful for preparing the SAP of the surface crosslinked SAP particles include all processes customarily used to synthesize SAPs, as described, for example, in Chapter 3 of "Modem Superabsorbent Polymer Technology," F.L Buchholz and A.T. Graham, Wiley-VCH (1998). A suitable process for polymerizing the acrylic acid is aqueous solution polymerization, wherein an aqueous solution containing acrylic acid and polymerization initiator is subjected to a polymerization reaction and a crosslinking reaction by the addition of an internal crosslinking agent, such as methylenebisacrylamide.

As previously noted, the polymerization reaction proceeds rapidly to yield a highly viscous hydrogel that is extruded, for example, onto a flat surface such as a continuously moving conveyor belt. The SAP hydrogel then is comminuted, and, if necessary, neutralized with a suitable base, for example, sodium carbonate, to provide SAP hydrogel particles having a degree of neutralization (DN) of about 25% to about 100%, preferably about 50% to about 85%, more preferably about 65% to about 80%.

After neutralization, the viscous SAP hydrogel particles are dehydrated (i.e., dried) to obtain SAP particles in a solid or powder form. The dehydration step can be performed, for example, by heating the viscous SAP hydrogel particles at a temperature of about 120°C for about 1 to about 2 hours in a forced-air oven or by heating the viscous hydrogel overnight at a temperature of about 60°C. The dried SAP particles then are surface crosslinked with a surface crosslinking agent, in the presence of a clay, as described below.

A preferred SAP is neutralized poly(acrylic acid), i.e., PAA. One nonlimiting method of manufacturing SAP particles is disclosed in U.S. Patent No. 6,187,828, incorporated herein by reference. A suitable PAA can be prepared as follows. Although this disclosure is directed primarily to the preparation of poly(acrylic acid) (i.e., PAA), other acidic and basic water-absorbing resins can be manufactured by identical or similar methods.

### EXAMPLE 1

In general, PAA can be prepared from an aqueous solution containing about 10% to about 40%, preferably about 15% to about 35%, by weight, acrylic acid, more preferably about 20% to about 30%, and most preferably about 25% to about 28% by weight, with an appropriate amount of internal crosslinking monomer. A PAA so obtained is neutralized with sodium carbonate, potassium carbonate, ammonium carbonate, sodium hydroxide, or a mixture thereof, to DN=60-95.

In particular, a solution containing 25% by weight acrylic acid, 0.07 mole percent methylenebisacrylamide, appropriate levels of initiators (2,2'-azobis(2-amidinopropane)dihydrochloride and sodium persulfate), at an initiator temperature of 18°C, yielded a hydrogel that, when neutralized with sodium carbonate powder to DN=75 percent and then dried, milled, sized, and post-modified by surface crosslinking, yielded a PAA with an average gel volume of 41.2 gm/gm, an absorption under load (AUL) of 34.1 gm/gm (0.28 psi load) and 27.1 gm/gm (0.7 psi load), a 7.7 weight percent extractables, and a residual acrylic acid content of 140 parts per million.

In accordance with an important feature of the present invention, it is not necessary to sift or sieve SAP fines from the dried, milled SAP particles because surface crosslinking in the presence of a clay reduces or essentially eliminates the SAP fines from the SAP. If necessary or desired, large particle size SAP particles, e.g., greater than 800 µm in diameter, can be sifted or sieved from the dry, milled SAP particles.

### SURFACE CROSSLINKER

SAP particles of the present invention are surface crosslinked. Surface crosslinking is achieved by contacting the SAP particles with a solution of a surface crosslinking agent to wet predominantly only the outer surfaces of the SAP particles. Surface crosslinking and drying of the SAP particles then is performed, preferably by heating at least the wetted surfaces of the SAP particles.

Typically, SAP particles are surface treated with a solution of a surface crosslinking agent. The solution contains about 0.01% to about 4%, and preferably about 0.4% to about 2%, by weight, surface crosslinking agent in a suitable solvent, for example, water or an alcohol. The solution can be applied as a fine spray onto the surface of freely tumbling SAP particles at a ratio of about 1:0.01 to about 1:0.5 parts by weight SAP particles to solution of surface crosslinking agent. To achieve the desired absorption properties, the surface crosslinking agent is distributed evenly on the surfaces of the SAP particles. For this purpose, mixing is performed in suitable mixers, e.g., fluidized bed mixers, paddle mixers, a rotating disc mixer, a ribbon mixer, a screw mixer, milling rolls, or twin-worm mixers.

The surface crosslinking agent is present in an amount of 0.001 % to about 5%, by weight of the surface crosslinked SAP particles, and preferably 0.01 % to about 2% by weight. To achieve the full advantage of the present invention, the surface crosslinking agent is present in an amount of about 0.05% to about 1% by weight of the surface crosslinked SAP particles.

The crosslinking reaction and drying of the surface-treated SAP particles are achieved by heating the surface-treated SAP particles at a suitable temperature, e.g., about 25°C to about 250°C, and preferably about 105°C to about 200°C, for about 60 to about 180, and preferably for about 60 to about 150 minutes. However, any other method of reacting the crosslinking agent to achieve surface crosslinking of the SAP particles, and any other method of drying SAP particles, such as microwave energy, can be used.

Surface treating with a surface crosslinking agent, and subsequent or simultaneous heating, provides additional polymer crosslinks in the vicinity of the surface of the SAP particles. The gradation of crosslinking from the surface of the SAP particles to interior, i.e., the anisotropy of crosslink density, can vary, both in depth and profile. Thus, for example, the depth of surface crosslinking can be shallow, with a relatively sharp transition from a high level to a low level of crosslinking. Alternatively, for example, the depth of surface crosslinking can be a significant fraction of the dimensions of the SAP particle, with a broader transition.

Surface crosslinking generally is performed after the final boundaries of the SAP particles are essentially established (e.g., by grinding, extruding, or foaming). However, it is also possible to effect surface crosslinking concurrently with the creation of final boundaries. Furthermore, some additional changes in SAP particle boundaries can occur even after surface crosslinks are introduced.

Suitable surface crosslinkers include, but are not limited to, di- or polyglycidyl compounds, such as diglycidyl phosphonates, ethylene glycol diglycidyl ether, and bischlorohydrin ethers of polyalkylene glycols; alkoxysilyl compounds; polyaziridines based on polyethers or substituted hydrocarbons, for example, bis-N-aziridinomethane; polyamines or polyamidoamines and their reaction products with epichlorohydrin; polyols, such as ethylene glycol, 1,2-propanediol, 1,4-butanediol, glycerol, methyltriglycol, polyethylene glycols having an average molecular weight M_{w} of 200-10,000, di- and polyglycerol, pentaerythritol, sorbitol, the ethoxylates of these polyols and their esters with carboxylic acids or carbonic acid such as ethylene carbonate or propylene carbonate; carbonic acid derivatives, such as urea, thiourea, guanidine, dicyandiamide, 2-oxazolidinone and its derivatives, bisoxazoline, polyoxazolines, di- and polyisocyanates; di- and poly-N-methylol compounds such as, for example, methylenebis(N-methylolmethacrylamide) or melamine-formaldehyde resins; compounds having two or more blocked isocyanate groups such as, for example, trimethylhexamethylene diisocyanate blocked with 2,2,6,6-tetramethylpiperidin-4-one.

Particularly suitable surface crosslinking agent include di- or polyglycidyl compounds, such as ethylene glycol diglycidyl ether. See U.S. Patent No. 6,159,591, incorporated herein by reference, for additional surface crosslinking agents for anionic and cationic SAPs, and method of surface crosslinking and annealing SAP particles.

With respect to an acidic or a basic water-absorbing resin, suitable surface crosslinking agents are capable of reacting with acid moieties or amino groups, and crosslinking the resin. Preferably, the surface crosslinking agent is alcohol soluble or water soluble, and possesses sufficient reactivity with the resin such that crosslinking occurs in a controlled fashion, preferably at a temperature of about 25°C to about 180°C.

Nonlimiting examples of suitable surface crosslinking agents for acidic resins include, but are not limited to:
(a) polyhydroxy compounds, such as glycols and glycerol;
(b) metal salts;
(c) quaternary ammonium compounds;
(d) a multifunctional epoxy compound;
(e) an alkylene carbonate, such as ethylene carbonate or propylene carbonate;
(f) a plyaziridine, such as 2,2-bishydroxymethyl butanol tris[3-(1-aziridine propionate]);
(g) a haloepoxy, such as epichlorohydrin;
(h) a polyamine, such as ethylenediamine;
(i) a polyisocyanate, such as 2,4-toluene dissocyanate;
(j) hydroxyalkylamides, hydroxyalkylamines, and oxazolinium ion, disclosed in U.S. Patent No. 6,376,618, U.S. Patent No. 6,391,451, and WO 01/8959, for example, bis[N,N-di(β-hydroxyethyl)]adipamide, available commercially as PRIMID™ XL-552, EMS-CHEMIE, Dornet, Switzerland, bis[N,N-di(β-hydroxypropyl)]succinamide, bis[N,N-di(β-hydroxyethyl)]azelamide, bis[N ,N-di(β-hydroxypropyl)]adipamide, bis[N-methyl-N-(β-hydroxyethyl)]oxamide, and PRIMID™ QM-1260;
(k) other crosslinking agents for acidic water-absorbing resins known to persons skilled in the art.

Nonlimiting examples of suitable surface crosslinking agents for basic resins include, but are not imited to:
(a) dihalides and disulfonate esters, for example, compounds of the formula

   Y-(CH₂)ₚ-Y,

   wherein p is a number from 2 to 12, and Y, independently, is halo (preferably bromo), tosylate, mesylate, or other alkyl, or aryl sulfonate esters;
(b) multifunctional aziridines;
(c) multifunctional aldehydes, for example, glutaraldehyde, trioxane, paraformaldehyde, terephthaldehyde, malonaldehyde, and glyoxal, and acetals and bisulfites thereof;
(d) halohydrins, such as epichlorohydrin;
(e) multifunctional epoxy compounds, for example, ethylene glycol diglycidyl ether, bisphenol A diglycidyl ether, and bisphenol F diglycidyl ether,
(f) multifunctional carboxylic acids and esters, acid chlorides, and anhydrides derived therefrom, for example, di- and polycarboxylic acids containing 2 to 12 carbon atoms, and the methyl and ethyl esters, acid chlorides, and anhydrides derived therefrom, such as oxalic acid, adipic acid, succinic acid, dodecanoic acid, malonic acid, and glutaric acid, and esters, anhydrides, and acid chlorides derived therefrom;
(g) organic titanates, such as TYZOR® AA, available from E.I. DuPont de Nemours, Wilmington, DE;
(h) melamine resins, such as the CYMEL® resins available from Cytec Industries, Wayne, NJ;
(i) hydroxymethyl ureas, such as N, N'-dihydroxymethyl-4,5-dihydroxyethylene urea;
(j) multifunctional isocyanates, such as toluene diisocyanate, isophorone diisocyanate, methylene diisocyanate; and
(k) other crosslinking agents for basic water-absorbing resins known to persons skilled in the art.

The following example illustrates surface crosslinking of SAP particles. In the following example, the SAP particles are lightly crosslinked PAA, neutralized about 75% to about 80% with sodium hydroxide.

### EXAMPLE 2

A solution containing 1% to 5%, by weight, of PRIMID™ XL-552 and 0% to 37.5%, by weight, propylene glycol in water was applied to the surface of SAP particles, at the rate of about 4 to about 10 grams of solution per 100 grams of SAP particles. The surface-treated SAP particles then were heat treated at about 150°C to about 170°C for about 60 to about 120 minutes. Excellent results were achieved using a 3.5% PRIMID™ XL-552/25% propylene glycol solution applied at about 7 grams of solution per 100 grams of SAP particles, then heat treating for about 120 minutes at 160°C.

### CLAY

A clay useful in the present surface crosslinked SAP particles can be a swelling or a nonswelling clay. Swelling clays have the ability to absorb water and are swellable, layered organic materials. Suitable swelling clays include, but are not limited to, montmorillonite, saponite, nontronite, laponite, beidelite, hectorite, sauconite, stevensite, vermiculite, volkonskoite, magadite, medmontite, kenyaite, and mixtures thereof.

Preferably, the swelling clay is a smectite or vermicullite clay. More preferably, the clay is a smectite clay. Examples of suitable smectites include, but are not limited to, montmorillonite (often referred to as bentonite), beidelite, nontronite, hectorite, saponite, sauconite, and laponite. Bentonite is a naturally occurring combination of clay particles, rich in montmorillonite and also including other smectites, as well as nonclay mineral constituents.

Suitable nonswelling clays include, without limitation, kaolin minerals (including kaolinite, dickite, and nacrite), serpentine minerals, mica minerals (including illite), chlorite minerals, sepolite, palygorskite, bauxite, and mixtures thereof.

The clay also can be an organophilic clay. As used here and hereafter, the term "organophilic" is defined as the property of a compound to absorb at least its own weight, and preferably many times its own weight, of an organic, water-immiscible compound. An organophilic compound optionally can absorb water or a water-miscible compound.

The terms "organophilic clay" and "organoclay" are used interchangeably herein to refer to various types of clay, e.g., smectites, that have organoammonium ions substituted for metal cations (e.g., sodium and/or potassium) present between the clay layers. The term "organoammonium ion" refers to a substituted ammonium ion wherein one or more hydrogen atoms are replaced by an aliphatic or aromatic organic group. The organoclays, therefore, are solid compounds that have an inorganic component and an organic component.

The preferred clay substrates of an organophilic clay are the smectite-type clays, particularly smectite-type clays that have a cation exchange capacity of at least 75 milliequivalents per 100 grams of clay. Useful clay substrates include, but are not limited to, the naturally occurring Wyoming variety of bentonite and similar clays, and hectorite, which is a magnesium-lithium silicate clay. The clays preferably first are converted to the sodium form if they are not already in this form. This conversion can be effected by a cation exchange reaction using a soluble sodium compound by methods well known in the art. Smectite-type clays prepared synthetically also can be utilized, for example, montmorillonite, bentonite, beidelite, hectorite, saponite, and stevensite. Other useful clay substrates include nontronite, illite, attapulgite, and a fuller's earth.

Organoclays useful in the present invention also include those set forth in Hauser U.S. Patent No. 2,531,427, incorporated herein by reference. These organoclays are modified clays that exhibit, in an inorganic liquid, some of the properties an untreated clay exhibits in water. For example, the ability to swell in organic liquids and form stable gels and colloidal dispersions.

Generally, the organoammonium ion substituted onto the clay substrate has an organic group that ranges from an aliphatic hydrocarbon moiety having 1 to 24 carbon atoms to an aromatic organic moiety, such as a benzyl group that can have a variety of groups substituted on the phenyl ring. The number of benzyl versus aliphatic hydrocarbon moieties substituted on the ammonium ion can vary from 3 to 0 aromatic moieties per aliphatic moiety (i.e., dimethyl dioctadecyl 0:2, methyl benzyl dioctadecyl 1:2, dibenzyl dioctabenzyl 2:2, tribenzyl octadecyl 3:1, and methyl dibenzyl octadecyl 2:1). The amount of organoammonium ion substituted onto the clay substrate typically is about 0.5% to about 50%, by weight of the organophilic clay.

Preferred organoclays comprise one or more of the following types of organoammonium cation-modified montmorillonite clays: 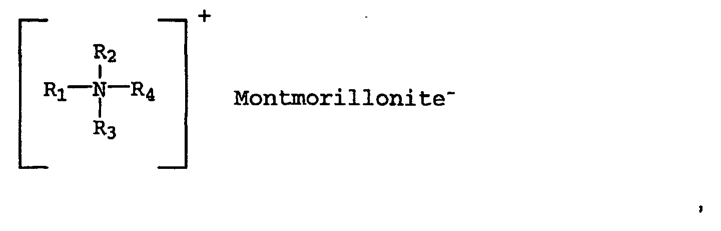 wherein R₁ is an alkyl group having at least 20, and up to, for example, 24 carbon atoms, and preferably having a chain length of 12 to 18 carbon atoms; R₂ is hydrogen, benzyl, or an alkyl group having at least 10, and up to, for example, 24 carbon atoms, and preferably 12 to 18 carbon atoms; and R₃ and R₄, independently, are a lower alkyl group, i.e., an alkyl group containing carbon chains of 1 to 4 atoms, and preferably methyl groups.

Other useful organoclays include benzyl organoclays, such as dimethyl benzyl (hydrogenated tallow) ammonium bentonite; methyl benzyl di(hy-drogenated tallow) ammonium bentonite; and more generally organoammonium-cation modified montmorillonite clays represented by the formula: 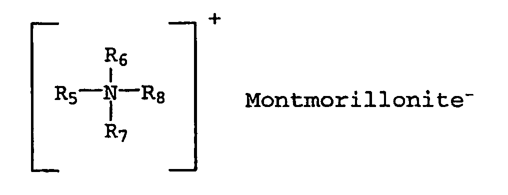 wherein R₅ is CH₃ or C₆H₅CH₂; R₆ is C₆H₅CH₂; and R₇ and R₈, independently, are alkyl groups containing long chain alkyl radicals having 14 to 22 carbon atoms, and most preferably where 20% to 35% of said long chain alkyl radicals contain 16 carbon atoms and 60% to 75% of said long chain alkyl radicals contain 18 carbon atoms.

The montmorillonite clays that can be so modified are the principal constituents of bentonite rock, and have the chemical compositions and characteristics described, for example, in Berry & Mason, "Mineralogy," pp. 508-509 (1959). Modified montmorillonite clays of this type (i.e., organoclays) are commercially available from Southern Clay Products, Inc., Gonzales, Texas, under trade designations such as CLAYTONE® 34 and 40, and from NL Industries, Inc., New York, NY, under trade designations such as BENTONE® 27, 34, and 38. Other organoclays useful in the invention are the higher dialkyl dimethyl ammonium organoclays, such as dimethyl di-(hydrogenated tallow) ammonium bentonite; the benzyl ammonium organoclays, such as dimethyl benzyl (hydrogenated tallow) ammonium bentonite; and ethylhydroxy ammonium organoclays, such as methyl bis(2-hydroxyethyl) octadecyl ammonium bentonite. Examples of nonswelling organophilic clays are bentonite clays treated with an amine containing three to eight carbon atoms, e.g., propylamine, butylamine, or octylamine.

Other commercially available clays include ULTRAGLOSS® clay (hydrous kaolin) from Engelhard Corporation, Iselin, NJ; Purified Clay from Nanocor Technologies, Arlington Heights, IL; and HYDROGLOSS® from Huber, Atlanta, GA.

A clay does not perform like an SAP with respect to absorbing and retaining large amounts of an aqueous fluid. A clay typically is referred to, and considered, as a diluent for SAP particles in an attempt to improve one or more properties of the SAP. It also is expected that other SAP properties would be adversely affected by diluting an SAP with a clay. However, it was found that after adding about 12% to about 35%, by weight, of a clay to SAP particles during a surface crosslinking step, the beneficial properties associated with an SAP were not diminished while the problems associated with SAP fines are improved or overcome.

The present invention, therefore, is directed to an improved method of manufacturing an SAP by introducing a sufficient amount of a clay to SAP particles during a surface crosslinking step. The improvements are realized in reducing or essentially eliminating the generation and recycling of SAP fines. SAP production, therefore, is increased and costs are reduced.

The following examples illustrate two nonlimiting embodiments of the present invention.

### EXAMPLE 3

In this example, SAP particles, including SAP fines, were surface crosslinked in the presence of a kaolin clay slurry. In particular, unsifted PAA (DN=73) (1000 grams) in a Lodige Model M5B coater machine was sprayed with a mixture containing water (21 grams), propylene glycol (21 grams), a kaolin clay slurry (286 grams, 70% active), and ethylene glycol diglycidyl ether (2 grams).

The surface treated SAP particles contained 20% boaa (based on weight of acrylic acid) kaolin clay, and 0.2% boaa ethylene glycol diglycidyl ether. The coating machine is configured with a jacket for heating the surface treated SAP particles. Coating time was about one minute. The temperature of both the SAP particles and the mixture was 25°C. After complete addition of the mixture, the surface treated SAP particles were heated to 120°C over a 30-minute period. After reaching 120°C, the surface treated SAP particles were heated for one additional hour to effect surface crosslinking. The resulting surface crosslinked SAP particles have a clay positioned in the vicinity of the SAP particle surfaces.

In this example, the starting PAA contained about 20% by weight SAP of SAP fines (i.e., <200 micron diameter). In the normal manufacturing process, an additional 5% by weight of SAP fines is removed from the product after surface crosslinking (i.e., 25% by weight total SAP fines are removed). In the above Example 3, only 4% by weight SAP fines were removed from the finished product. This results in the elimination/reduction of about 80% to 85% of the SAP fines that typically would be recycled back into the SAP manufacturing process.

Example 3 shows that a present SAP manufacturing process, which generates 25% SAP fines, can be substantially improved with respect to SAP throughout and process economics by the addition of a clay to the SAP particles during a surface crosslinking step. The reduction or essential elimination of the amount of SAP fines increases SAP production capacity because only a small amount of recycled SAP fines must be dried a second time. In addition, the substantial cost associated with sifting SAP particles to remove SAP fines, recycling the SAP fines, and control of the recycling process is reduced or eliminated.

The following example shows that performance benefits also result from surface crosslinking SAP particles free of SAP fines in the presence of a clay.

### EXAMPLE 4

In this example, an SAP containing 80 wt% PAA (DN=73), 20 wt% sodium silicate, and free of SAP fines was surface crosslinked in the presence of a clay as follows. First, the following mixtures were prepared: water (21 gms), propylene glycol (21 gms), kaolin clay slurry as in Example 3 (143 gms (10%), 246 gms (20%), or 429 gms (30% boaa)), and ethylene glycol diglycidyl ether (2 gms (0.2%) or 3 gms (0.3% boaa)).

The mixtures then were applied to the SAP to provide SAP particles surface crosslinked with 0.2% or 0.3%, by weight, ethylene glycol diglycidyl ether (boaa), and containing 10%, 20%, or 30%, by weight, kaolin clay (boaa) in the vicinity of the SAP particle surfaces. Surface treating and surface crosslinking was performed as set forth in Example 3.

The resulting surface-crosslinked SAP particles exhibited about a 10% performance improvement over identical surface-crosslinked SAP particles lacking a clay for typically measured properties, such as AUL and CRC. The surface-crosslinked particles of the present invention also exhibited a substantial increase in SFC, i.e., from about 20 x 10⁷ cm³sec/g to about 100 x 10⁷ cm³sec/g. Such a result is surprising for SAP particles containing 20% sodium silicate and 20% kaolin clay, for a total of 40%, by weight, diluent in the SAP. The surface-treated SAP particles of Example 4, therefore, are more economical to prepare because they contain a high percentage of diluent, while surprisingly providing improved SAP particle performance.

The following examples and data illustrate the effect of adding a clay to an SAP during a surface crosslinking step on the absorbent properties of the SAP particles. In the test results set forth herein, the surface crosslinked SAP were tested for absorption under load at and 0.9 psi (AUL (0.9 psi) and AUL (0.7 psi)) after one hour. Absorption under load (AUL) is a measure of the ability of an SAP to absorb fluid under an applied pressure. The AUL was determined by the following method.

An SAP (0.160 g+/-0.001 g) is carefully scattered onto a 140 micron, water-permeable mesh attached to the base of a hollow plexiglas cylinder with an internal diameter of 25 mm. The sample is covered with a 100 g cover plate and the cylinder assembly weighed. This gives an applied pressure of 20 g/cm² (0.3 psi). Alternatively, the sample can be covered with a 250 g or a 300 g cover plate to give an applied pressure of 51 g/cm² (0.7 psi) or 66 g/cm³ (0.9 psi). The screened base of the cylinder is placed in a 100 mm petri dish containing 25 milliliters of a test solution (usually 0.9% saline), and the polymer is allowed to absorb for 1 hour. By reweighing the cylinder assembly, the AUL (at a given pressure) is calculated by dividing the weight of liquid absorbed by the dry weight of polymer before liquid contact.

The CRC (centrifuge retention capacity) test is designed to measure the amount of saline solution retained inside SAP particles subjected to a specific centrifugal force. The measurement of CRC is disclosed in U.S. Patent No. 6,187,828 and U.S. Patent No. 5,633,316, each incorporated herein by reference.

Another important property of SAP particles is permeability when swollen with a liquid to form a hydrogel zone or layer, as defined by the Saline Flow Conductivity (SFC) value of the composition particles. SFC measures the ability of the composition to transport saline fluids, such as the ability of the hydrogel layer formed from the swollen composition to transport body fluids. A material having relatively high SFC value is an air-laid web of woodpulp fibers. Typically, an air-laid web of pulp fibers (e.g., having a density of 0.15 g/cc) exhibits an SFC value of about 200 x 10⁻⁷ cm³sec/g. In contrast, typical hydrogel-forming SAPs exhibit SFC values of 1 x 10⁻⁷ cm³sec/g or less. A method for determining the SFC value of SAP particles is set forth in U.S. Patent No. 5,599,335, incorporated herein by reference.

The acquisition time/rewet under pressure test is performed using laboratory pads. To produce these laboratory pads, 11.2 g of cellulose fluff and 13.0 g of SAP particles are homogeneously fluidized in an air box, and, by application of a slight vacuum, laid down on a mold 12 by 26 cm in size. This mixture then is wrapped in tissue paper, and compressed two times for 15 seconds each under a pressure of 200 bar. A laboratory pad so produced is attached to a horizontal surface. The center of the pad is determined and marked. Saline solution (0.9 wt% NaCl) is applied through a plastic plate having a ring in the middle (internal diameter of ring:6.0 cm, height:4.0 cm). The plastic plate is loaded with additional weights such that the total load on the pad is 13.6 g/cm². The plastic plate is placed on the pad such that the center of the pad is also the center of the application ring. Saline solution (80 ml) is applied three times. The salt solution is measured in a measuring cylinder, and applied in one portion to the pad through the ring in the plate. Simultaneously, the time is measured until the solution has completely penetrated into the pad. The measured time is recorded as Acquisition Time 1. Thereafter, the pad is weighted with a plate for 20 minutes, the load further being maintained at 13.6 g/cm². Thereafter, the plate is removed, 10 g ± 0.5 g of filter paper (Schleicher & Schuell, 1450 CV) is placed on the central spot and loaded with a weight (area 10 cm x 10 cm, weight 3.5 kg) for 15 seconds. After this period, the weight is removed and the filter paper is reweighed. The weight difference is noted as Rewet 1. Thereafter, the plastic plate with application ring again is placed on the pad and the saline is applied for a second time. The time measured is noted as Acquisition Time 2. The procedure is repeated as described, but 45 g ± 0.5 g of filter paper are used for the Rewet test. Rewet 2 is noted. The same method is employed to determine Acquisition Time 3. Filter paper (50 g ± 0.5 g) is used to determine Rewet 3.

Surprisingly it has been found that the absorption and retention properties of SAP particles can be improved by adding a clay to SAP particles during surface crosslinking of the SAP particles. The resulting surface crosslinked SAP particles containing clay in the vicinity of the surfaces of the SAP particles exhibit improved fluid acquisition rates, and improved fluid permeability (i.e., SFC) through fluid swollen SAP hydrogel particles.

In addition, the presence of a clay in the vicinity of the surfaces of the SAP particles substantially reduces the generation and recycling of SAP fines, which facilitates manufacture, and especially drying, of SAP hydrogel particles, and provides SAP particles that are easier to handle during production of absorbent articles. As an additional benefit, clay present on the surfaces of dried SAP particles act as a processing aid, which facilitates handling of the SAP particles in the manufacture of absorbent articles, especially in humid environments. These features allow a reduction in the production time and cost of SAP particles, with a corresponding improvement in the amount of SAP produced per unit time.

It is theorized, but not relied upon herein, that adding a clay to SAP particles during a surface crosslinking step agglomerates SAP fines and reduces or essentially eliminates recycling of SAP fines by addition to an SAP hydrogel. The surface crosslinked SAP particles of the present invention also exhibit improved acquisition rates, permeability, and dry feel after fluid absorption.

The following examples and test results illustrate the new and unexpected results achieved by the incorporation of a clay onto a surface of SAP particles during a surface-crosslinking step. As illustrated in the following examples and tests, the present invention provides SAP particles having improved fluid acquisition rates and permeability of a fluid through swollen SAP particles.

### EXAMPLE 5

A 10 L capacity polyethylene vessel, well insulated by foamed polymer material, was charged with 3400 g demineralized water and 1400 g acrylic acid. Pentaerythritol triallyl ether (12.6 g) and a 15% strength by weight aqueous solution of a polyamidoamine-epichlorhydrin adduct (75 g) (RETEN® 204 LS from Hercules) then were added as internal crosslinking monomers. At a temperature of 10°C, 2,2'-azobisamidinopropane dihydrochloride (2.2 g) dissolved in 25 g demineralized water, and potassium peroxodisulfate (4 g) dissolved in 150 g of demineralized water, were added in succession with stirring. The resulting solution was deoxygenated by bubbling a nitrogen stream through the solution for 30 minutes, followed by the addition of ascorbic acid (0.4 g) dissolved in 25 g demineralized water. The reaction solution then was allowed to stand without stirring, and the temperature of the polymerization rose to about 95°C. A solid SAP hydrogel was obtained, which subsequently mechanically comminuted, and adjusted to pH 6.0 by addition of 50% strength by weight sodium hydroxide solution. The hydrogel then was dried, ground, and classified to a particle size distribution <850 µm. The amount of SAP particles <150 µm was 18% by weight. The SAP particles (1 kg) then were sprayed in a plowshare mixer with a solution containing 1,2-propylene glycol (25 g) in distilled water (25 g), ethylene glycol diglycidyl ether (2.0 g), and varying amounts of a clay slurry (ULTRA WHITE® 90 clay slurry from Engelhard Industries, containing 70 wt% of clay), followed by heating at 140°C for two hours. Absorbing properties and particle size distribution of the surface-crosslinked SAP particles were as follows:

| **Amount of clay (in wt%) based on dry powder** | **CRC* (g/g)** | **AUL 0.9 psi* (g/g)** | **SFC* (cm**^{**3**}**sec/g)** | **Particles <200 µm (wt%)** | **Particles >800 µm (wt%)** |
|---|---|---|---|---|---|
| 0 | 20.5 | 19.6 | 50 x 10⁻⁷ | 21 | 0.6 |
| 5 | 20.4 | 19.8 | 55 x 10⁻⁷ | 20 | 1.2 |
| 10 | 20.7 | 19.2 | 60 x 10⁻⁷ | 16 | 0.9 |
| 15 | 20.3 | 19.5 | 110 x 10⁻⁷ | 4.5 | 1.6 |
| 20 | 20.8 | 19.5 | 155 x 10⁻⁷ | 2.7 | 1.2 |
| 25 | 19.9 | 18.9 | 185 x 10⁻⁷ | 1.7 | 2.0 |
| 30 | 18.2 | 17.8 | 220 x 10⁻⁷ | 1.5 | 4.4 |
| 35 | 17.4 | 16.9 | 250 x 10⁻⁷ | 1.2 | 7.8 |

| | | | | | |
|---|---|---|---|---|---|
| * Properties measured after classification to 150-800 µm. | | | | | |

### COMPARATIVE EXAMPLE 1

Example 5 was repeated, except the SAP hydrogel was dried, ground, and classified to a particle size distribution 150-850 µm. The SAP particles (1 kg) was sprayed in a plowshare mixer with a solution containing 1,2-propylene glycol (25 g), distilled water (25 g), and ethylene glycol diglycidyl ether (2.0 g), followed by heating at 140°C for two hours. No clay was added to the SAP hydrogels during the surface crosslinking step. Absorbing properties of the surface crosslinked SAP particles were as follows:

| | |
|---|---|
| CRC | = 20.3 g/g |
| AUL (0.9 psi) | = 19.8 g/g |
| SFC | = 75 x 10⁻⁷ cm³sec/g. |

Example 5 and Comparative Example 1 show that the addition of a clay to SAP particles during a surface crosslinking step substantially reduces the amount of SAP fines (i.e., <200 µm diameter) without adversely affecting the fluid absorption and retention properties of the SAP.

### EXAMPLE 6

An aqueous monomer mixture containing 25 wt% acrylic acid, 0.6 wt% ethoxylated trimethylolpropane triacrylate based on acrylic acid (boaa), 0.28 wt% sodium persulfate boaa, 0.075 wt% DAROCURE® 1173 boaa, and 0.025 wt% IR-GACURE® 651 was prepared and cooled to 12°C. The resulting monomer mixture then was polymerized for 12.5 minutes under a UV light (UV intensity=20 mW/cm²). The resulting PAA hydrogel was extruded through a KitchenAid Model K5SS mixer fitted with a meat grinder attachment Next, sodium carbonate was added to the hydrogel to neutralize the acrylic acid groups to 75 mol% followed by two additional extrusions. The hydrogel was dried at 150°C for one hour, then milled and sized to <800 µm. The amount of SAP particles <200 µm in diameter was 21 wt%. The dry SAP particles then were surface crosslinked by spraying a solution containing 0.1 wt% ethylene glycol diglycidyl ether based on powder, 1.65 wt% propylene glycol based on powder, 3.33 wt% distilled water based on powder, and varying amounts of a clay slurry (ULTRA WHITE® 90 clay slurry containing 70 wt% clay) onto the SAP particles, followed by heating at 150°C for one hour. The absorbing properties of the resulting surface crosslinked SAP particles were as follows:

| **Amount of clay (in wt%) based on dry powder** | **CRC* (g/g)** | **AUL 0.7 psi* (g/g)** | **AUL 0.7 psi* (g/g)** | **Particles <200 µm (wt%)** | **Particles >800 µm (wt%)** |
|---|---|---|---|---|---|
| 0 | 29.2 | 29.9 | 23.4 | 26 | 1.3 |
| 5 | 28.9 | 29.5 | 23.2 | 24 | 1.2 |
| 10 | 28.5 | 29.3 | 22.8 | 20 | 1.5 |
| 15 | 28.1 | 29.0 | 22.4 | 5 | 1.6 |
| 20 | 27.1 | 28.7 | 22.0 | 3 | 1.8 |
| 25 | 26.8 | 28.3 | 21.5 | 2 | 2.0 |
| 30 | 25.1 | 26.9 | 20.1 | 2 | 3.5 |
| 35 | 23.4 | 24.1 | 17.8 | 2 | 5.8 |

| | | | | | |
|---|---|---|---|---|---|
| * Properties measured after classification to 200-800 µm. | | | | | |

The weight amount of SAP fines was substantially reduced by the addition of a clay during the surface crosslinking step, without an adverse effect on SAP performances.

The surface crosslinked SAP particles, after classification to 200-800 µm (i.e., SAP fines were sifted from the SAP particles), also were tested using the acquisition time/rewet under pressure test:

| **Amount of clay (in wt%) based on dry particles** | **Acquisition Time 1 (seconds)** | **Acquisition Time 2 (seconds** | **Acquisition Time 3 (seconds)** | **Rewet 1 (grams)** | **Rewet 2 (grams)** | **Rewet 3 (grams)** |
|---|---|---|---|---|---|---|
| 0 | 28 | 89 | 143 | <0.1 | 0.6 | 3.0 |
| 5 | 29 | 86 | 139 | <0.1 | 0.6 | 2.9 |
| 10 | 27 | 79 | 132 | <0.1 | 0.7 | 3.2 |
| 15 | 24 | 65 | 118 | <0.1 | 0.6 | 2.9 |
| 20 | 21 | 59 | 98 | <0.1 | 0.7 | 3.3 |
| 25 | 18 | 55 | 85 | 0.1 | 0.9 | 3.1 |
| 30 | 17 | 52 | 80 | 0.3 | 1.3 | 4.4 |
| 35 | 19 | 58 | 78 | 0.6 | 1.8 | 7.5 |

The above table shows that the present surface-crosslinked SAP particles, in addition to essentially eliminating the problem of SAP fines, perform well even in the presence of high amounts of a clay.

The above test results show that the surface crosslinked SAP particles of the present invention can be used to absorb aqueous fluids. The fluid can be a body fluid, an industrial waste, or any other fluid that one desires to absorb. The absorbed fluid can be any water-containing fluid, and typically contains electrolytes, for example, urine, blood, saline, menses, and similar liquids.

The SAP particles are especially useful in absorbent articles, such as diapers, adult incontinence products, tampons, and sanitary napkins. The present SAP particles, therefore, are useful in personal hygiene articles comprising:
(A) a fluid-pervious topsheet;
(B) a fluid-impervious backsheet;
(C) a core positioned between (A) and (B), said core comprising:
   (C1) about 10% to 100% by weight of the surface-crosslinked SAP particles of the present invention, and
   (C2) 0% to about 90% by weight of a fiber material;
(D) optionally one or more tissue layers positioned directly above and/or below said core (C); and
(E) optionally an acquisition layer positioned between (A) and (C).

The fluid-pervious topsheet (A) is the layer which is in direct contact with the skin of the wearer. Topsheet (A) generally comprises synthetic or cellulosic fibers or films, i.e., polyesters, polyolefins, rayon, or natural fibers, such as cotton. In the case of nonwoven materials, the fibers generally are joined together by binders such as a polyacrylate. Preferred materials are polyesters, rayon and blends thereof, polyethylene, and polypropylene. The fluid-impervious layer (B) is generally a sheet of polyethylene or polypropylene.

The core (C) includes SAP particles (C1) of the present invention, and also can include a fiber material (C2). Fiber material (C2) typically is hydrophilic, i.e., aqueous fluids are rapidly distributed across the fibers. The fiber material typically is cellulose, modified cellulose, rayon, or a polyester, such as polyethylene terephthalate. Preferred fibers are cellulose fibers, such as pulp. The fibers generally have a diameter of about 1 to about 200 µm, preferably about 10 to about 100 µm, and a minimum length of about 1 mm.

The amount of fiber material (C2) based on the total weight of the core is typically about 20% to about 80% by weight, preferably about 40% to about 70% by total weight of C(1) and C(2). Core (C) typically also can be a heavily loaded core (e.g., 60-95 wt% SAP particles/5-40 wt % fluff).

The SAP particles often are present in core (C) as a pressed sheet containing the particles, and optionally fluff and/or nonwoven fibers. A single absorbent layer or sheet containing SAP particles of the present invention can be used as the absorbent component of a core (C). Preferably, a plurality of absorbent layers or sheets are used in the core (C), more preferably together with a wicking layer (e.g., a tissue layer) between absorbent layers or sheets to provide improved wicking of a fluid between and through the absorbent sheets. In more preferred embodiments, at least one of the absorbent layers or sheets in a core (C) contains nonwoven fibers to improve wet strength of the absorbent core and assist in wicking.

A preferred core (C) contains two to five absorbent layers or sheets. By utilizing a laminate of thin absorbent layers or sheets, as opposed to a single, thicker absorbent layer or sheet, horizontal expansion of the core is decreased, and vertical expansion is promoted. This feature provides a good fluid transport through the core, provides a better fitting diaper after an initial insult, and avoids leaking when the diaper is subsequently rewet by a second and additional insult. In more preferred embodiments, core (C) contains a laminate of two or more absorbent layers or sheets of SAP particles wherein a wicking layer is positioned between each absorbent sheet layer or sheet, and on top and at the bottom of the laminate.

An absorbent layer or sheet containing surface crosslinked SAP particles of the present invention, or a laminate comprising such layers or sheets, is present in an absorbent core to provide a desired basis weight (i.e., weight of SAP in the core) of about 50 to about 800 gsm (grams/square meter), and preferably about 150 to about 600 gsm. To achieve the full advantage of the present invention, the basis weight is about 300 to about 550 gsm. The desired basis weight of the core is related to the end use of the core. For example, diapers for newborns have a low basis weight, as opposed to a medium basis weight for toddlers, and a high basis weight for overnight diapers.

In a preferred embodiment, a present diaper core consists essentially of a topsheet (A), a core (C), and a backsheet (B), i.e., an acquisition layer is not present. An example of a topsheet (A) is staple length polypropylene fibers having a denier of about 1.5, such as Hercules-type 151 polypropylene marketed by Hercules, Inc., Wilmington, DE. As used herein, the term "staple length fibers" refers to fibers having a length of at least about 15.9 mm (0.62 inches). The backsheet (B) is impervious to liquids, and typically is manufactured from a thin plastic film, although other flexible liquid impervious materials also can be used. The backsheet prevents exudates absorbed and contained in the absorbent core (C) from wetting articles, such as bed sheets and undergarments, that contact the diaper.

For an absorbent article having a core (C) containing a "fluff" component, the "fluff" comprises a fibrous material in the form of a web or matrix. Fibers include naturally occurring fibers (modified or unmodified). Examples of suitable unmodified/modified naturally occurring fibers include cotton, Esparto grass, bagasse, kemp, flax, silk, wool, wood pulp, chemically modified wood pulp, and jute. See WO 98/37149 and U.S. Patent No. 5,859,074, each incorporated herein by reference, for a complete discussion of "fluff components for use in an absorbent sheet article.

The cores also can include an optional nonwoven fiber, for example, polypropylene, polyethylene, polyethylene terephthalate, viscose, and mixtures thereof. Also, an open fiber mesh of nonwoven fibers can be used, for example, cellulose acetate fiber. Nonwoven fibers can be made by drylaid thermobonded, carded air-through bonded, spunbond, or spun-meltblown-spun processes. Nonwoven fibers impart additional wet strength to an absorbent layer or sheet when used in an amount of about 10 to about 20 grams per square meter (gsm) of sheet material.

Suitable fibers, and fiber meshes, can be made from polyvinyl chloride, polyvinyl fluoride, polytetrafluoroethylene, polyvinylidene chloride, polyacrylics such as ORLON®, polyvinyl acetate, polyethylvinyl acetate, nonsoluble or soluble polyvinyl alcohol, polyolefins such as polyethylene (e.g., PULPEX®) and polypropylene, polyamides (e.g., nylon), polyesters (e.g., DACRON® or KODEL®), polyurethanes, polystyrenes, and the like.

Hydrophilic fibers are preferred, and include rayon, polyester fibers, such as polyethylene terephthalate (e.g., DACRON®), hydrophilic nylon (e.g., HY-DROFIL®), and the like. Suitable hydrophilic fibers can also be obtained by hydrophilizing hydrophobic fibers, such as surfactant-treated or silica-treated thermoplastic fibers derived from, for example, polyolefins, such as polyethylene or polypropylene, polyacrylics, polyamides, polystyrenes, polyurethanes, and the like.

The improved results demonstrated by a core containing surface crosslinked SAP particles of the present invention permit the thickness of the core to be reduced. Typically, cores contain 50% or more fluff or pulp to achieve rapid liquid absorption while avoiding problems like gel blocking. The present cores, which contain SAP particles, acquire liquids sufficiently fast to avoid problems, like gel blocking, and, therefore, the amount of fluff or pulp in the core can be reduced, or eliminated. A reduction in the amount of the low-density fluff results in a thinner core, and, accordingly, a thinner diaper. Therefore, a core of the present invention can contain at least 50% SAP particles, preferably at least 60%, and up to 80% of the SAP particles. In various embodiments, the presence of a fluff is no longer necessary, or desired.

Many modifications and variations of the invention as hereinbefore set forth can be made without department from the spirit and scope thereof, and, therefore, only such limitations should be imposed as are indicated by the appended claims.

## Claims

1. Surface-crosslinked superabsorbent particles comprising:
(i) 50% to 88%, by weight, of a superabsorbent polymer, said superabsorbent polymer comprising 0.001% to 5%, by weight, of a surface crosslinking agent;
(ii) 12% to 35%, by weight, of a day, said clay present in the vicinity of surfaces of the superabsorbent particles; and
(iii) 0% to 25%, by weight, of an inorganic network builder,
said particles prepared by a method comprising the steps of:
(a) polymerizing one or more monomers capable of providing a superabsorbent absorbent polymer in the presence of an internal crosslinking monomer to form a superabsorbent polymer hydrogel;
(b) comminuting the superabsorbent polymer hydrogel to form superabsorbent polymer hydrogel particles;
(c) drying the superabsorbent polymer hydrogel particles of step (b) to provide dry superabsorbent particles;
(d) applying a mixture comprising a surface crosslinking agent and a clay to the surface of the superabsorbent particles of step (c) to provide surface-treated superabsorbent polymer particles; and
(e) then heating the surface-treated superabsorbent polymer particles for a sufficient time at a sufficient temperature to surface crosslink the surface-treated superabsorbent polymer particles and position the clay in the vicinity of the surfaces of the surface-crosslinked superabsorbent particles.

2. The particles of claim 1 wherein the superabsorbent polymer is present in an amount of 55% to 85%, by weight, and the clay is present in an amount of 15% to 25%, by weight.

3. The particles of one of the claims 1 or 2 wherein the inorganic network builder is present in an amount of 5% to 20%, by weight.

4. The particle of one of the claims 1 to 3 wherein the inorganic network builder is selected from the group consisting of a silicate, an aluminate, and an aluminosilicate.

5. The particle of one of the claims 1 to 4 wherein the inorganic network builder comprises sodium silicate, sodium aluminate, or a mixture thereof.

6. The particles of one of the claims 1 to 5 wherein less than 5%, by weight, of the particles have a diameter of 200 *µ*M or less.

7. The particles of one of the claims 1 to 6 wherein the superabsorbent polymer comprises a polymerized α,β-unsaturated carboxylic acid, or salt or anhydride thereof.

8. The particles of one of the claims 1 to 6 wherein the monomer is selected from the group consisting of acrylic acid, methacrylic acid, ethacrylic acid, α-chloroacrylic acid, α-cyanoacrylic acid, β-methylacrylic acid, α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, fumaric acid, tricarboxyethylene, maleic anhydride, vinyl sulfonic acid, allyl sulfonic acid, vinyl toluene sulfonic acid, styrene sulfonic acid, sulfoethyl acrylate, sulfoethyl methacrylate, sulfopropyl acrylate, sulfopropyl methacrylate, sulfopropyl acrylate, sulfopropyl methacrylate, 2-hydroxy-3-methacryloxypropyl sulfonic acid, 2-acrylamido-2-methylpropane sulfonic acid, methacryloxy ethyl phosphate, and mixtures thereof.

9. The particles of one of the claims 1 to 6 wherein the superabsorbent polymer is selected from the group consisting of poly(acrylic acid), a hydrolyzed starch-acrylonitrile graft copolymer, a starch-acrylic acid graft copolymer, a saponified vinyl acetate-acrylic ester copolymer, a hydrolyzed acrylonitrile copolymer, a hydrolyzed acrylamide copolymer, an ethylene-maleic anhydride copolymer, an isobutylene-maleic anhydride copolymer, a poly(vinylsulfonic add), a poly(vinylphosphonic acid), a poly(vinylphosphoric add), a poly(vinylsulfuric acid), a sulfonated polystyrene, and salts and mixtures thereof.

10. The particles of one of the claims 1 to 6 wherein the superabsorbent polymer is selected from the group consisting of a poly(vinylamine), a poly(dialkylaminoalkyl (meth)acrylamide), a polyethylenimine, a poly(allylamine), a poly(allylguanidine), a poly(di-methyldiallylammonium hydroxide), a quatemized polystyrene derivative, a guanidine-modified poly-styrene, a quatemized poly((meth)acrylamide) or ester analog, a poly(vinylguanidine), and salts and mixtures thereof.

11. The particles of one of the claims 1 to 6 wherein the superabsorbent polymer comprises polyacrylic acid neutralized 25% to 100%.

12. The particles of one of the claims 1 to 11 wherein the clay is a swelling clay selected from the group consisting of montmorillonite, saponite, nontronite, laponite, beidelite, hectorite, sauconite, stevensite, vermiculite, volkonskoite, magadite, medmontite, kenyaite, and mixtures thereof.

13. The particles of one of the claims 1 to 11 wherein the clay is a nonswelling clay selected from the group consisting of a kaolin mineral, a serpentine mineral, a mica mineral, a chlorite mineral, sepolite, palygorskite, bauxite, and mixtures thereof.

14. The particles of claim 13 wherein the nonswelling clay comprises a kaolinite.

15. The particles of one of the claims 1 to 11 wherein the clay is an organophilic clay having an organic component and an inorganic component.

16. The particles of claim 15 wherein the inorganic component of the organophilic clay comprises smectite, bentonite, hectorite, montmorillonite, beidelite, saponite, stevensite, nontronite, illite, attapulgite, a zeolite, fuller's earth, and mixtures thereof.

17. The particles of one of the claims 15 or 16 wherein the inorganic component of the organophilic day comprises montmorillonite.

18. The particles of one of the claims 15 to 17 wherein the organic component of the organophilic day comprises 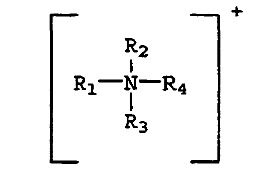 wherein R₁ is an alkyl group having at least 20 carbon atoms, R₂ is hydrogen, benzyl, or an alkyl group having at least 10 carbon atoms, and R₃ and R₄, independently, are a lower alkyl group; 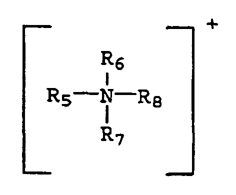 wherein R₅ is CH₃ or C₆H₅CH₂, R₆ is C₆H₅CH₂, and R₇ and R₈, independently, are alkyl groups containing long chain alkyl radicals having 14 to 22 carbon atoms; or a mixture thereof.

19. The particles of one of the claims 15 to 18 wherein the organophilic clay is selected from the group consisting of dimethyl benzyl (hydrogenated tallow) ammonium bentonite, methyl benzyl di(hydrogenated tallow) ammonium bentonite, dimethyl di(hydrogenated tallow) ammonium bentonite, methyl bis(2-hydroxyethyl) octadecyl ammonium bentonite, a bentonite clay treated with an amine containing three to eight carbon atoms, and mixtures thereof.

20. A method of absorbing an aqueous medium comprising contacting the medium with the superabsorbent particles of one of the claims 1 to 19.

21. The method of claim 20 wherein the aqueous medium contains electrolytes.

22. The method of claim 21 wherein the electrolyte-containing aqueous medium is selected from the group consisting of urine, saline, menses, and blood.

23. An absorbent article comprising the superabsorbent particles of one of the claims 1 to 19.

24. The article of claim 23 wherein the article is a diaper or a catamenial device.

25. A diaper having a core, said core comprising at least 10% by weight of the superabsorbent polymer of one of the claims 1 to 19.

26. The diaper of claim 25 further comprising a topsheet in contact with a first surface of the core, and a backsheet in contact with a second surface of the core, said second core surface opposite from said first core surface.

27. The diaper of claim 26 further comprising an acquisition layer disposed between the topsheet and the core.

28. A method of manufacturing surface-crosslinked superabsorbent polymer particles comprising a water-absorbing resin and 12% to 35%, by weight, of a clay distributed in the vicinity of surfaces of the particles comprising the steps of:
(a) forming an aqueous monomer mixture comprising (i) at least one monomer capable of forming a superabsorbent polymer, (ii) an internal crosslinking agent, and (iii) a polymerization catalyst;
(b) polymerizing the monomer in the aqueous mixture to form a superabsorbent polymer hydrogel;
(c) comminuting the superabsorbent polymer hydrogel to provide superabsorbent polymer hydrogel particles;
(d) drying the superabsorbent polymer hydrogel particles for a sufficient time at a sufficient temperature to provide dry superabsorbent polymer particles;
(e) applying a mixture of a clay and a surface crosslinking agent to surfaces of the dry superabsorbent polymer particles to provide surface-treated superabsorbent polymer particles; and
(f) heating the surface treated superabsorbent polymer particles for a sufficient time at a sufficient temperature to surface crosslink the surface treated superabsorbent polymer particles and position the clay in the vicinity of the surfaces of the particles.

29. The method of claim 28 wherein the monomer capable of forming the superabsorbent polymer is selected from the group consisting of acrylic acid, methacrylic acid, ethacrylic acid, α-chloroacrylic acid, α-cyanoacrylic acid, β-methylacrylic acid, α-phenylacrylic acid, β-acryloxypropionic acid, sorbic acid, α-chlorosorbic acid, angelic acid, cinnamic acid, p-chlorocinnamic acid, β-stearylacrylic acid, itaconic acid, citraconic acid, mesaconic acid, glutaconic acid, aconitic acid, maleic acid, fumaric acid, tricarboxyethylene, maleic anhydride, vinyl sulfonic acid, allyl sulfonic acid, vinyl toluene sulfonic acid, styrene sulfonic acid, sulfoethyl acrylate, sulfoethyl methacrylate, sulfopropyl acrylate, sulfopropyl methacrylate, sulfopropyl acrylate, sulfopropyl methacrylate, 2-hydroxy-3-methacryloxypropyl sulfonic acid, 2-acrylamide-2-methylpropane sulfonic acid, methacryloxy ethyl phosphate, and mixtures thereof.

30. The method of claim 28 wherein the superabsorbent polymer is selected from the group consisting of poly(acrylic acid), a hydrolyzed starch-acrylonitrile graft copolymers, a starch-acrylic acid graft copolymer, a saponified vinyl acetate-acrylic ester copolymer, a hydrolyzed acrylonitrile copolymer, a hydrolyzed acrylamide copolymer, an ethylene-maleic anhydride copolymer, an isobutylenemaleic anhydride copolymer, poly(vinylsulfonic acid), poly(vinylphosphonic acid), poly(vinylphosphoric acid), poly(vinylsulfuric acid), sulfonated polystyrene, a poly(vinylamine), a poly(dialkylaminoalkyl (meth)acrylamide), a lightly crosslinked polyethylenimine, a poly(allylamine), a poly(allylguanidine), a poly(dimethyldiallylammonium hydroxide), a quatemized polystyrene derivative, a guanidine-modified polystyrene, a quatemized poly((meth)acrylamide) or ester analog, and mixtures thereof.

## Patentansprüche

1. Oberflächlich vernetzte superabsorbierende Teilchen, enthaltend:
(i) 50 bis 88 Gew.-% eines Superabsorbers, enthaltend 0,001 bis 5 Gew.-% eines Oberflächenvernetzungsmittels,
(ii) 12 bis 35 Gew.-% eines Tons, wobei der Ton in den superabsorbierenden Teilchen oberflächennah enthalten ist, und
(iii) 0 bis 25 Gew.-% eines anorganischen Netzbildners,
wobei die Herstellung der Teilchen nach einem Verfahren erfolgt, bei dem man
(a) mindestens ein zu einem Superabsorber polymerisierbares Monomer in Gegenwart eines intern vernetzend wirkenden Monomers zu einem Superabsorberhydrogel polymerisiert,
(b) das Superabsorberhydrogel zu Superabsorberhydrogelteilchen zerkleinert,
(c) die Superabsorberhydrogelteilchen aus Schritt (b) zu trockenen superabsorbierenden Teilchen trocknet,
(d) auf die superabsorbierenden Teilchen aus Schritt
(c) oberflächlich eine ein Oberflächenvernetzungsmittel und einen Ton enthaltende Mischung aufbringt und so oberflächlich behandelte Superabsorberteilchen erhält, und
(e) anschließend die oberflächlich behandelten Superabsorberteilchen entsprechend lange bei einer entsprechenden Temperatur trocknet, um die oberflächlich behandelten Superabsorberteilchen oberflächlich zu vernetzen und den Ton bezüglich der oberflächlich vernetzten superabsorbierenden Teilchen oberflächennah zu positionieren.

2. Teilchen nach Anspruch 1, bei denen der Superabsorber in einer Menge von 55 bis 85 Gew.-% und der Ton in einer Menge von 15 bis 25 Gew.-% enthalten ist.

3. Teilchen nach einem der Ansprüche 1 oder 2, bei denen der anorganische Netzbildner in einer Menge von 5 bis 20 Gew.-% enthalten ist.

4. Teilchen nach einem der Ansprüche 1 bis 3, bei denen der anorganische Netzbildner unter Silicat, Aluminat und Alumosilicat ausgewählt ist.

5. Teilchen nach einem der Ansprüche 1 bis 4, bei denen der anorganische Netzbildner wenigstens teilweise aus Natriumsilicat, Natriumaluminat oder einer Mischung davon besteht.

6. Teilchen nach einem der Ansprüche 1 bis 5, bei denen weniger als 5 Gew.-% der Teilchen einen Durchmesser von höchstens 200 µm aufweisen.

7. Teilchen nach einem der Ansprüche 1 bis 6, bei denen der Superabsorber eine □,□-ungesättigte Carbonsäure oder deren Salz oder Anhydrid einpolymerisiert enthält.

8. Teilchen nach einem der Ansprüche 1 bis 6, bei denen das Monomer unter Acrylsäure, Methacrylsäure, Ethacrylsäure, □-Chloracrylsäure, □-Cyanacrylsäure, □-Methylacrylsäure, □-Phenylacrylsäure, □-Acryloxypropionsäure, Sorbinsäure, □-Chlorsorbinsäure, Angelicasäure, Zimtsäure, p-Chlorzimtsäure, □-Stearylacrylsäure, Itaconsäure, Citraconsäure, Mesaconsäure, Glutaconsäure, Aconitsäure, Maleinsäure, Fumarsäure, Tricarboxyethylen, Maleinsäureanhydrid, Vinylsulfonsäure, Allylsulfonsäure, Vinyltoluolsulfonsäure, Styrolsulfonsäure, Sulfoethylacrylat, Sulfoethylmethacrylat, Sulfopropylacrylat, Sulfopropylmethacrylat, 2-Hydroxy-3-methacryloxypropylsulfonsäure, 2-Acrylamid-2-methylpropansulfonsäure, Methacryloxyethylphosphat und deren Mischungen ausgewählt ist.

9. Teilchen nach einem der Ansprüche 1 bis 6, bei denen der Superabsorber aus der Gruppe: Polyacrylsäure, hydrolysiertes Stärke-Acrylnitril-Pfropfcopolymerisat, Stärke-Acrylsäure-Pfropfcopolymerisat, verseiftes Vinylacetat-Acrylester-Copolymerisat, hydrolysiertes Acrylnitrilcopolymerisat, hydrolysiertes Acrylamidcopolymerisat, Ethylen-Maleinsäureanydrid-Copolymerisat, Isobutylen-Maleinsäureanhydrid-Copolymerisat, Polyvinylsulfonsäure, Polyvinylphosphonsäure, Polyvinylphosphorsäure, Polyvinylschwefelsäure, sulfoniertes Polystyrol und Salze und Mischungen davon ausgewählt ist.

10. Teilchen nach einem der Ansprüche 1 bis 6, bei denen der Superabsorber aus der Gruppe: Polyvinylamin, Polydialkylaminoalkyl(meth)acrylamid, Polyethylenimin, Polyallylamin, Polyallylguanidin, Polydimethyldiallylammoniumhydroxid, quaternisiertes Polystyrolderivat, guanidinmodifiziertes Polystyrol, quaternisiertes Poly(meth)acrylamid oder Esteranalog, Polyvinylguanidin und Salze und Mischungen davon ausgewählt ist.

11. Teilchen nach einem der Ansprüche 1 bis 6, bei denen der Superabsorber wenigstens teilweise aus einer zu 25% bis 100% neutralisierten Polyacrylsäure besteht.

12. Teilchen nach einem der Ansprüche 1 bis 11, bei denen es sich bei dem Ton um einen unter Montmorrillonit, Saponit, Nontronit, Laponit, Beidelit, Hektorit, Saukonit, Stevensit, Vermikulit, Volkonskoit, Magadit, Medmontit, Kenyait und deren Mischungen ausgewählten Quellton handelt.

13. Teilchen nach einem der Ansprüche 1 bis 11, bei denen es sich bei dem Ton um einen unter Kaolinmineral, Serpentinmineral, Glimmermineral, Chloritmineral, Sepolit, Palygorskit, Bauxit und deren Mischungen ausgewählten Quickton handelt.

14. Teilchen nach Anspruch 13, bei denen der Quickton wenigstens teilweise aus einem Kaolinit besteht.

15. Teilchen nach einem der Ansprüche 1 bis 11, bei denen es sich bei dem Ton um einen organophilen Ton mit einer organischen Komponente und einer anorganischen Komponente handelt.

16. Teilchen nach Anspruch 15, bei denen die anorganische Komponente des organophilen Tons wenigstens teilweise aus Smektit, Bentonit, Hektorit, Montmorrillonit, Beidelit, Saponit, Stevensit, Nontronit, Illit, Attapulgit, einem Zeolith, Bleicherde und deren Mischungen besteht.

17. Teilchen nach einem der Ansprüche 15 oder 16, bei denen die anorganische Komponente des organophilen Tons wenigstens teilweise aus Montmorrillonit besteht.

18. Teilchen nach einem der Ansprüche 15 bis 17, bei denen die anorganische Komponente des organophilen Tons wenigstens teilweise aus 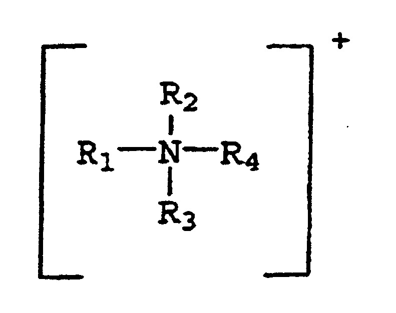 wobei R₁ eine Alkylgruppe mit mindestens 20 Kohlenstoffatomen, R₂ Wasserstoff, Benzyl oder eine Alkylgruppe mit mindestens 10 Kohlenstoffatomen und R₃ und R₄ unabhängig voneinander eine Niederalkylgruppe bedeuten; 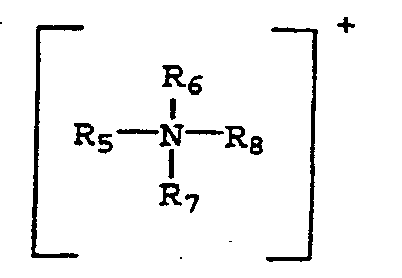 wobei R₅ CH₃ oder C₆H₅CH₂, R₆ C₆H₅CH₂ und R₇ und R₈ unabhängig voneinander langkettige Alkylreste mit 14 bis 22 Kohlenstoffatomen enthaltende Alkylgruppen bedeuten, oder einer Mischung davon besteht.

19. Teilchen nach einem der Ansprüche 15 bis 18, bei denen der organophile Ton aus der Gruppe: Dimethylbenzyl(hydrotalg)ammoniumbentonit, Methylbenzyldi(hydrotalg)ammoniumbentonit, Dimethyldi(hydrotalg)ammoniumbentonit, Methylbis(2-hydroxyethyl)octadecylammoniumbentonit, mit einem drei bis acht Kohlenstoffatome enthaltenden Amin behandelter Bentonitton und deren Mischungen ausgewählt ist.

20. Verfahren zur Absorption eines wäßrigen Mediums, bei dem man das Medium mit den superabsorbierenden Teilchen gemäß einem der Ansprüche 1 bis 19 in Berührung bringt.

21. Verfahren nach Anspruch 20, bei dem das wäßrige Medium Elektrolyte enthält.

22. Verfahren nach Anspruch 21, bei dem das elektrolythaltige wäßrige Medium unter Urin, Kochsalzlösung, Blut und Menstruationsblut ausgewählt wird.

23. Absorbierendes Erzeugnis mit den superabsorbierenden Teilchen gemäß einem der Ansprüche 1 bis 19.

24. Erzeugnis nach Anspruch 23, bei dem es sich um eine Windel oder um ein Monatshygieneprodukt handelt.

25. Windel mit Kern, bei der der Kern zu mindestens 10 Gew.-% aus dem Superabsorber gemäß einem der Ansprüche 1 bis 19 besteht.

26. Windel nach Anspruch 25, bei der zusätzlich eine an einer ersten Oberfläche des Kerns anliegende Oberlage und eine an einer der ersten Oberfläche des Kerns gegenüberliegenden zweiten Oberfläche anliegende Unterlage enthalten sind.

27. Windel nach Anspruch 26, bei der zusätzlich eine zwischen Oberlage und Kern angeordnete Akquisitionsschicht enthalten ist.

28. Verfahren zur Herstellung von oberflächlich vernetzten Superabsorberteilchen, enthaltend ein wasserabsorbierendes Harz und 12 bis 35 Gew.-% eines oberflächennah verteilten Tons, bei dem man
(a) eine wäßrige Monomermischung bereitstellt, die (i) minestens ein zu einem Superabsorber polymerisierbares Monomer, (ii) ein intern vernetzend wirkendes Monomer und (iii) einen Polymerisationskatalysator enthält,
(b) das Monomer in der wäßrigen Mischung zu einem Superabsorberhydrogel polymerisiert,
(c) das Superabsorberhydrogel zu Superabsorberhydrogelteilchen zerkleinert,
(d) die Superabsorberhydrogelteilchen entsprechend lange bei einer entsprechenden Temperatur trocknet, um trockene Superabsorberteilchen zu erhalten,
(e) auf die trockenen Superabsorberteilchen oberflächlich eine Mischung von einem Ton und einem Oberflächenvernetzungsmittel aufbringt und so oberflächlich behandelte Superabsorberteilchen erhält und
(f) die oberflächlich behandelten Superabsorberteilchen entsprechend lange bei einer entsprechenden Temperatur erhitzt, um die oberflächlich behandelten Superabsorberteilchen oberflächlich zu vernetzen und den Ton bezüglich der Teilchen oberflächennah zu positionieren.

29. Verfahren nach Anspruch 28, bei dem das zum Superabsorber polymerisierbare Monomer unter Acrylsäure, Methacrylsäure, Ethacrylsäure, α-Chloracrylsäure, α-Cyanacrylsäure, β-Methylacrylsäure, α-Phenylacrylsäure, β-Acryloxypropionsäure, Sorbinsäure, α-Chlorsorbinsäure, Angelicasäure, Zimtsäure, p-Chlorzimtsäure, β-Stearylacrylsäure, Itaconsäure, Citraconsäure, Mesaconsäure, Glutaconsäure, Aconitsäure, Maleinsäure, Fumarsäure, Tricarboxyethylen, Maleinsäureanhydrid, Vinylsulfonsäure, Allylsulfonsäure, Vinyltoluolsulfonsäure, Styrolsulfonsäure, Sulfoethylacrylat, Sulfoethylmethacrylat, Sulfopropylacrylat, Sulfopropylmethacrylat, 2-Hydroxy-3-methacryloxypropylsulfonsäure, 2-Acrylamid-2-methylpropansulfonsäure, Methacryloxyethylphosphat und deren Mischungen ausgewählt wird.

30. Verfahren nach Anspruch 28, bei dem der Superabsorber aus der Gruppe: Polyacrylsäure, hydrolysiertes Stärke-Acrylnitril-Pfropfcopolymerisat, Stärke-Acrylsäure-Pfropfcopolymerisat, verseiftes Vinylacetat-Acrylester-Copolymerisat, hydrolysiertes Acrylnitrilcopolymerisat, hydrolysiertes Acrylamidcopolymerisat, Ethylen-Maleinsäureanydrid-Copolymerisat, Isobutylen-Maleinsäureanhydrid-Copolymerisat, Polyvinylsulfonsäure, Polyvinylphosphonsäure, Polyvinylphosphorsäure, Polyvinylschwefelsäure, sulfoniertes Polystyrol, Polyvinylamin, Polydialkylaminoalkyl(meth)acrylamid, leicht vernetztes Polyethylenimin, Polyallylamin, Polyallylguanidin, Polydimethyldiallylammoniumhydroxid, quaternisiertes Polystyrolderivat, guanidinmodifiziertes Polystyrol, quaternisiertes Poly(meth)acrylamid oder Esteranalog und Mischungen davon ausgewählt wird.

## Revendications

1. Particules superabsorbantes réticulées en surface, comprenant :
(i) 50% à 88% en poids d'un polymère superabsorbant, ledit polymère superabsorbant comprenant 0,001% à 5% en poids d'un agent de réticulation en surface;
(ii) 12% à 35% en poids d'une argile, ladite argile étant présente à proximité de surfaces des particules superabsorbantes; et
(iii) 0% à 25% en poids d'un adjuvant de réseau inorganique;
lesdites particules étant préparées par un procédé comprenant les étapes suivantes :
(a) on polymérise un ou plusieurs monomères capables de former un polymère absorbant superabsorbant en présence d'un monomère de réticulation interne pour former un hydrogel polymère superabsorbant;
(b) on broie l'hydrogel polymère superabsorbant pour former des particules d'hydrogel polymère superabsorbant;
(c) on sèche les particules d'hydrogel polymère superabsorbant de l'étape (b) pour obtenir des particules superabsorbantes sèches;
(d) on applique un mélange comprenant un agent de réticulation en surface et une argile à la surface des particules superabsorbantes de l'étape (c) pour obtenir des particules de polymère superabsorbant traitées en surface; et
(e) ensuite, on chauffe les particules de polymère superabsorbant traitées en surface pendant une période de temps suffisante à une température suffisante pour réticuler en surface les particules de polymère superabsorbant traitées en surface et positionner l'argile à proximité des surfaces des particules de polymère superabsorbant réticulées en surface.

2. Particules selon la revendication 1, dans lesquelles le polymère superabsorbant est présent en quantité de 55% à 85% en poids et l'argile est présente en quantité de 15% à 25% en poids.

3. Particules selon l'une quelconque des revendications 1 ou 2, dans lesquelles l'adjuvant de réseau inorganique est présent en quantité de 5% à 20% en poids.

4. Particules selon l'une quelconque des revendications 1 à 3, dans lesquelles l'adjuvant de réseau inorganique est choisi dans le groupe constitué d'un silicate, d'un aluminate et d'un aluminosilicate.

5. Particules selon l'une quelconque des revendications 1 à 4, dans lesquelles l'adjuvant de réseau inorganique comprend le silicate de sodium, l'aluminate de sodium ou un de leurs mélanges.

6. Particules selon l'une quelconque des revendications 1 à 5, dans lesquelles une proportion de moins de 5% en poids des particules a un diamètre de 200 µm ou moins.

7. Particules selon l'une quelconque des revendications 1 à 6, dans lesquelles le polymère superabsorbant comprend un acide carboxylique polymérisé à insaturation α, β ou un de ses sels ou anhydrides.

8. Particules selon l'une quelconque des revendications 1 à 6, dans lesquelles le monomère est choisi dans le groupe constitué des éléments suivants : acide acrylique, acide méthacrylique, acide éthacrylique, acide α-chloro-acrylique, acide α-cyanoacrylique, acide β-méthylacrylique, acide α-phénylacrylique, acide β-acryloxypropionique, acide sorbique, acide α-chlorosorbique, acide angélique, acide cinnamique, acide p-chlorocinnamique, acide β-stéarylacrylique, acide itaconique, acide citraconique, acide mésaconique, acide glutaconique, acide aconitique, acide maléique, acide fumarique, tricarboxyéthylène, anhydride maléique, acide vinylsulfonique, acide allylsulfonique, acide vinyltoluènesulfonique, acide styrènesulfonique, acrylate de sulfoéthyle, méthacrylate de sulfoéthyle, acrylate de sulfopropyle, méthacrylate de sulfopropyle, acide 2-hydroxy-3-méthacryloxypropylsulfonique, acide 2-acrylamido-2-méthylpropanesulfonique, phosphate de méthacryloxyéthyle et leurs mélanges.

9. Particules selon l'une quelconque des revendications 1 à 6, dans lesquelles le polymère superabsorbant est choisi dans le groupe constitué des éléments suivants : acide poly(acrylique), copolymère greffé d'amidon hydrolysé et d'acrylonitrile, copolymère greffé d'amidon et d'acide acrylique, copolymère saponifié d'acétate de vinyle et d'ester acrylique, copolymère d'acrylonitrile hydrolysé, copolymère d'acrylamide hydrolysé, copolymère d'éthylène et d'anhydride maléique, copolymère d'isobutylène et d'anhydride maléique, acide poly(vinylsulfonique), acide poly(vinylphosphonique), acide poly(vinylphosphorique), acide poly(vinylsulfurique), polystyrène sulfoné et leurs sels et leurs mélanges.

10. Particules selon l'une quelconque des revendications 1 à 6, dans lesquelles le polymère superabsorbant est choisi dans le groupe constitué des éléments suivants : poly(vinylamine), poly(dialkylaminoalkyl(méth)acrylamide), polyéthylèneimine, poly(allylamine), poly(allylguanidine), poly(hydroxyde de diméthyldiallylammonium), dérivé de polystyrène quaternisé, polystyrène modifié par une guanidine, poly((méth)acrylamide) quaternisé ou analogue d'ester, poly(vinylguanidine) et leurs sels et leurs mélanges.

11. Particules selon l'une quelconque des revendications 1 à 6, dans lesquelles le polymère superabsorbant comprend de l'acide polyacrylique neutralisé à 25% à 100%.

12. Particules selon l'une quelconque des revendications 1 à 11, dans lesquelles l'argile est une argile gonflante choisie dans le groupe constitué des éléments suivants : montmorillonite, saponite, nontronite, laponite, beidellite, hectorite, sauconite, stevensite, vermiculite, volkonskoïte, magadite, medmontite, kenyaïte et leurs mélanges.

13. Particules selon l'une quelconque des revendications 1 à 11, dans lesquelles l'argile est une argile non gonflante choisie dans le groupe constitué des éléments suivants : minéral de kaolin, minéral de serpentine, minéral de mica, minéral de chlorite, sépiolite, palygorskite, bauxite et leurs mélanges.

14. Particules selon la revendication 13, dans lesquelles l'argile non gonflante comprend une kaolinite.

15. Particules selon l'une quelconque des revendications 1 à 11, dans lesquelles l'argile est une argile organophile ayant un composant organique et un composant inorganique.

16. Particules selon la revendication 15, dans lesquelles le composant inorganique de l'argile organophile comprend la smectite, la bentonite, l'hectorite, la montmorillonite, la beidellite, la saponite, la stevensite, la nontronite, l'illite, l'attapulgite, une zéolite, l'argile à foulon et leurs mélanges.

17. Particules selon l'une quelconque des revendications 15 ou 16, dans lesquelles le composant inorganique de l'argile organophile comprend la montmorillonite.

18. Particules selon l'une des revendications 15 à 17, dans lesquelles le composant organique de l'argile organophile comprend le corps de formule : 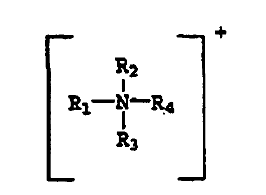 où R₁ est un groupement alkyle ayant au moins 20 atomes de carbone, R₂ est l'hydrogène, un benzyle ou un groupement alkyle ayant au moins 10 atomes de carbone, et R₃ et R₄ sont indépendamment un groupement alkyle inférieur; 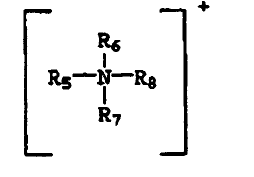 où R₅ est CH₃ ou C₆H₅CH₂, R₆ est C₆H₅CH₂ et R₇ et R₈ sont indépendamment des groupements alkyle contenant des radicaux alkyle à longue chaîne ayant 14 à 22 atomes de carbone; ou un de leurs mélanges.

19. Particules selon l'une quelconque des revendications 15 à 18, dans lesquelles l'argile organophile est choisie dans le groupe constitué des éléments suivants : bentonite de diméthylbenzyl-(suif hydrogéné)ammonium, bentonite de méthylbenzyl-di(suif hydrogéné)ammonium, bentonite de diméthyl-di(suif hydrogéné)ammonium, bentonite de méthyl-bis(2-hydroxyéthyl)octadécylammonium, argile de bentonite traitée avec une amine contenant trois à huit atomes de carbone, et leurs mélanges.

20. Procédé d'absorption d'un milieu aqueux comprenant la mise en contact du milieu avec les particules superabsorbantes d'une des revendications 1 à 19.

21. Procédé selon la revendication 20, dans lequel le milieu aqueux contient des électrolytes.

22. Procédé selon la revendication 21, dans lequel le milieu aqueux contenant des électrolytes est choisi dans le groupe constitué des éléments suivants : urine, solution physiologique, menstrues et sang.

23. Article absorbant comprenant les particules superabsorbantes de l'une quelconque des revendications 1 à 19.

24. Article selon la revendication 23, dans lequel l'article est une couche ou un dispositif cataménial.

25. Couche ayant une partie centrale, ladite partie centrale comprenant au moins 10% en poids du polymère superabsorbant de l'une quelconque des revendications 1 à 19.

26. Couche selon la revendication 25, comprenant en outre une feuille supérieure en contact avec une première surface de la partie centrale et une feuille de fond en contact avec une seconde surface de la partie centrale, ladite seconde surface de la partie centrale étant opposée à ladite première surface de la partie centrale.

27. Couche selon la revendication 26, comprenant en outre une couche d'acquisition disposée entre la feuille supérieure et la partie centrale.

28. Procédé de fabrication de particules de polymère superabsorbant réticulées en surface, comprenant une résine absorbant l'eau et 12% à 35% en poids d'une argile distribuée à proximité de surfaces des particules, comprenant les étapes suivantes :
(a) on forme un mélange aqueux de monomère(s) comprenant (i) au moins un monomère capable de former un polymère superabsorbant, (ii) un agent de réticulation interne et (iii) un catalyseur de polymérisation;
(b) on polymérise le monomère dans le mélange aqueux pour former un hydrogel polymère superabsorbant;
(c) on broie l'hydrogel polymère superabsorbant pour former des particules d'hydrogel polymère superabsorbant;
(d) on sèche les particules d'hydrogel polymère superabsorbant pendant une période de temps suffisante à une température suffisante pour obtenir des particules sèches de polymère superabsorbant;
(e) on applique un mélange d'une argile et d'un agent de réticulation en surface à des surfaces des particules sèches de polymère superabsorbant pour obtenir des particules de polymère superabsorbant traitées en surface; et
(f) on chauffe les particules de polymère superabsorbant traitées en surface pendant une période suffisante à une température suffisante pour réticuler en surface les particules de polymère superabsorbant traitées en surface et positionner l'argile à proximité des surfaces des particules.

29. Procédé selon la revendication 28, dans lequel le monomère capable de former le polymère superabsorbant est choisi dans le groupe constitué des éléments suivants : acide acrylique, acide méthacrylique, acide éthacrylique, acide α-chloroacrylique, acide α-cyanoacrylique, acide β-méthylacrylique, acide α-phénylacrylique, acide β-acryloxypropionique, acide sorbique, acide α-chlorosorbique, acide angélique, acide cinnamique, acide p-chlorocinnamique, acide β-stéarylacrylique, acide itaconique, acide citraconique, acide mésaconique, acide glutaconique, acide aconitique, acide maléique, acide fumarique, tricarboxyéthylène, anhydride maléique, acide vinylsulfonique, acide allylsulfonique, acide vinyltoluènesulfonique, acide styrènesulfonique, acrylate de sulfoéthyle, méthacrylate de sulfoéthyle, acrylate de sulfopropyle, méthacrylate de sulfopropyle, acide 2-hydroxy-3-méthacryloxypropylsulfonique, acide 2-acrylamide-2-méthylpropanesulfonique, phosphate de méthacryloxyéthyle et leurs mélanges.

30. Procédé selon la revendication 28, dans lequel le polymère superabsorbant est choisi dans le groupe constitué des éléments suivants : acide poly(acrylique), copolymères greffés d'amidon hydrolysé et d'acrylonitrile, copolymère greffé d'amidon et d'acide acrylique, copolymère saponifié d'acétate de vinyle et d'ester acrylique, copolymère d'acrylonitrile hydrolysé, copolymère d'acrylamide hydrolysé, copolymère d'éthylène et d'anhydride maléique, copolymère d'anhydride isobutylènemaléique, poly(acide vinylsulfonique), poly(acide vinylphosphonique), poly(acide vinylphosphorique), poly(acide vinylsulfurique), polystyrène sulfoné, poly(vinylamine), poly(dialkylaminoalkyl-(méth)acrylamide), polyéthylèneimine légèrement réticulée, poly(allylamine), poly(allylguanidine), poly(hydroxyde de diméthyldiallylammonium), dérivé de polystyrène quaternisé, polystyrène modifié à la guanidine, poly((méth)acrylamide) quaternisé ou analogue d'ester et leurs mélanges.
